(19) Europäisches Patentamt
European Patent Office
Office européen des brevets

(11) **EP 4 217 443 B1**

(12) **FASCICULE DE BREVET EUROPEEN**

(45) Date de publication et mention
de la délivrance du brevet:
**29.05.2024 Bulletin 2024/22**

(21) Numéro de dépôt: **21772806.2**

(22) Date de dépôt: **09.09.2021**

(51) Classification Internationale des Brevets (IPC):
*C10G 1/10* (2006.01)     *B09B 3/40* (2022.01)
*C10B 53/00* (2006.01)     *C10G 65/12* (2006.01)
*C10G 31/09* (2006.01)     *C10G 31/08* (2006.01)
*C10G 25/00* (2006.01)     *C10G 9/36* (2006.01)
*C10G 69/06* (2006.01)     *C10G 47/20* (2006.01)
*C10G 47/18* (2006.01)     *C10G 45/34* (2006.01)
*B09B 3/00* (2022.01)     *B09B 5/00* (2006.01)
*C10B 53/07* (2006.01)     *C10G 1/00* (2006.01)
*B09B 3/70* (2022.01)

(52) Classification Coopérative des Brevets (CPC):
**C10G 1/10; B09B 3/40; B09B 3/70; C10G 1/002;
C10G 9/36; C10G 25/00; C10G 31/08; C10G 31/09;
C10G 45/34; C10G 47/18; C10G 47/20;
C10G 65/12; C10G 69/06;** C10B 53/07;
C10G 2300/1003;        (Cont.)

(86) Numéro de dépôt international:
**PCT/EP2021/074824**

(87) Numéro de publication internationale:
**WO 2022/063597 (31.03.2022 Gazette 2022/13)**

(54) **PROCEDE DE TRAITEMENT D'HUILES DE PYROLYSE DE PLASTIQUES ET/OU DE COMBUSTIBLES SOLIDES DE RECUPERATION CHARGEES EN IMPURETES**

VERFAHREN ZUR AUFBEREITUNG VON PYROLYSEÖLEN AUS MIT VERUNREINIGUNGEN BELADENEN KUNSTSTOFFEN UND/ODER FESTEN RÜCKGEWONNENEN BRENNSTOFFEN

METHOD FOR PROCESSING PYROLYSIS OILS FROM PLASTICS AND/OR SOLID RECOVERED FUELS LOADED WITH IMPURITIES

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **25.09.2020 FR 2009750**

(43) Date de publication de la demande:
**02.08.2023 Bulletin 2023/31**

(73) Titulaire: **IFP Energies nouvelles
92500 Rueil-Malmaison (FR)**

(72) Inventeurs:
• **WEISS, Wilfried
92852 RUEIL-MALMAISON CEDEX (FR)**
• **QUIGNARD, Alain
92852 RUEIL-MALMAISON CEDEX (FR)**
• **NGUYEN-HONG, Duc
92500 RUEIL-MALMAISON (FR)**

(74) Mandataire: **IFP Energies nouvelles
Département Propriété Industrielle
Rond Point de l'échangeur de Solaize
BP3
69360 Solaize (FR)**

(56) Documents cités:
**WO-A1-2014/001632     FR-A1- 3 050 735
US-A1- 2019 161 683**

**(Cont. page suivante)**

(52) Classification Coopérative des Brevets (CPC):
(Cont.)
C10G 2300/1011; C10G 2300/202; C10G 2300/205;
Y02P 30/20

**Description**

**DOMAINE TECHNIQUE**

La présente invention concerne un procédé de traitement d'une huile de pyrolyse de plastiques et/ou de combustibles solides de récupération, chargée en impuretés afin d'obtenir un effluent hydrocarboné qui peut être valorisé en étant au moins en partie directement intégré à un pool naphta ou diesel ou comme charge d'une unité de vapocraquage. Plus particulièrement, la présente invention concerne un procédé de traitement d'une charge issue de la pyrolyse des déchets plastiques et/ou de CSR, afin d'éliminer au moins en partie des impuretés que ladite charge peut contenir en quantités importantes, et de manière à hydrogéner la charge pour pouvoir la valoriser.

**TECHNIQUE ANTERIEURE**

Les plastiques issus des filières de collecte et de tri peuvent subir une étape de pyrolyse afin d'obtenir entre autres des huiles de pyrolyse. Ces huiles de pyrolyse de plastiques sont généralement brûlées pour générer de l'électricité et/ou utilisées en tant que combustible dans des chaudières industrielles ou de chauffage urbain.

Les combustibles solides de récupération (CSR), aussi appelés « refuse derived fuel » ou RDF selon la terminologie anglo-saxonne, sont des déchets non dangereux solides préparés en vue d'une valorisation énergétique, qu'ils proviennent de déchets ménagers et assimilés, de déchets d'activités économiques ou de déchets de construction démolition. Les CSR sont généralement un mélange de n'importe quel déchet combustible tel que des pneus usés, des sous-produits alimentaires (graisses, farines animales, etc.), des déchets de viscose et de bois, des fractions légères issues de déchiqueteuses (par exemple de véhicules usagés, d'équipements électriques et électroniques (DEEE), des déchets ménagers et commerciaux, des résidus du recyclage de divers types de déchets, dont de certains déchets municipaux, les déchets plastiques, textiles, bois entre autres. Les CSR contiennent en général des déchets plastiques. Les CSR sont aujourd'hui surtout valorisés en énergie. Ils peuvent être directement utilisés comme substituts aux combustibles fossiles dans des installations de co-incinération (centrales thermiques à charbon et lignite, cimenteries, fours à chaux) ou dans des unités d'incinération des ordures ménagères, ou indirectement dans des unités de pyrolyse dédiées à la valorisation énergétique : les huiles de pyrolyse de CSR sont ainsi généralement brûlées pour générer de l'électricité, voire utilisées en tant que combustible dans des chaudières industrielles ou de chauffage urbain.

Une autre voie de valorisation des huiles de pyrolyse de plastiques ou de CSR est de pouvoir utiliser ces huiles de pyrolyse en tant que charge d'une unité de vapocraquage afin de (re)créer des oléfines, ces dernières étant des monomères constitutifs de certains polymères. Cependant, les déchets plastiques ou les CSR sont généralement des mélanges de plusieurs polymères, par exemple des mélanges de polyéthylène, de polypropylène, de polyéthylène téréphtalate, de polychlorure de vinyle, de polystyrène. De plus, en fonction des usages, les plastiques peuvent contenir, en plus des polymères, d'autres composés, comme des plastifiants, des pigments, des colorants ou encore des résidus de catalyseurs de polymérisation, ainsi que d'autres impuretés très variées, organiques et minérales, provenant des opérations de séparation des centres de tri, opération dont la sélectivité ne peut-être totale. Les huiles issues de la pyrolyse de plastiques ou de CSR comprennent ainsi beaucoup d'impuretés, en particulier des dioléfines, des métaux, du silicium, ou encore des composés halogénés, notamment des composés à base de chlore, des hétéroéléments comme du soufre, de l'oxygène et de l'azote, des insolubles, à des teneurs souvent élevées et incompatibles avec les unités de vapocraquage ou les unités situées en aval des unités de vapocraquage, notamment les procédés de polymérisation et les procédés d'hydrogénation sélective. Ces impuretés peuvent générer des problèmes d'opérabilité et notamment des problèmes de corrosion, de cokage ou de désactivation catalytique, ou encore des problèmes d'incompatibilité dans les usages des polymères cibles. La présence de dioléfines conduit très souvent à des problèmes d'instabilité de l'huile de pyrolyse se caractérisant par la formation de gommes. Les gommes et les insolubles éventuellement présents dans l'huile de pyrolyse peuvent générer des problèmes de colmatage dans les procédés.

De plus, lors de l'étape de vapocraquage, les rendements en oléfines légères recherchées pour la pétrochimie, notamment l'éthylène et le propylène, dépendent très fortement de la qualité des charges envoyées au vapocraquage. L'indice BMCI (Bureau of Mines Corrélation Index selon la terminologie anglo-saxonne) est souvent utilisé pour caractériser les coupes hydrocarbonées. Cet indice, développé pour les produits hydrocarbonés issus de pétroles bruts, est calculé à partir de la mesure de la masse volumique et de la température moyenne d'ébullition : il est égal à 0 pour une paraffine linéaire et à 100 pour le benzène. Sa valeur est donc d'autant plus élevée que le produit analysé à une structure condensée aromatique, les naphtènes ayant un BMCI intermédiaire entre les paraffines et les aromatiques. Globalement, les rendements en oléfines légères augmentent quand la teneur en paraffines augmente et donc quand le BMCI diminue. A l'inverse, les rendements en composés lourds non recherchés et/ou en coke augmentent quand le BMCI augmente.

Le document WO 2018/055555 propose un procédé de recyclage des déchets plastiques global, très général et relativement complexe, allant de l'étape même de pyrolyse des déchets plastiques jusqu'à l'étape de vapocraquage. Le procédé de la demande WO 2018/055555 comprend, entre autres, une étape d'hydrotraitement de la phase liquide issue directement de la pyrolyse, de préférence dans des conditions assez poussées notamment en termes de température, par exemple à une température comprise entre 260 et 300°C, une étape de séparation de l'effluent d'hydrotraitement puis une étape d'hydrodealkylation de l'effluent lourd séparé à une température de préférence élevée, par exemple comprise entre 260 et 400°C.

La demande de brevet non publiée FR20/01.758 décrit un procédé de traitement d'une huile de pyrolyse de plastiques, comprenant :

a) l'hydrogénation sélective de ladite charge en présence d'hydrogène et d'un catalyseur d'hydrogénation sélective pour obtenir un effluent hydrogéné ;

b) l'hydrotraitement en lit fixe dudit effluent hydrogéné en présence d'hydrogène et d'un catalyseur d'hydrotraitement, pour obtenir un effluent d'hydrotraitement ;

c) une séparation de l'effluent d'hydrotraitement en présence d'un flux aqueux, à une température entre 50 et 370°C, pour obtenir un effluent gazeux, un effluent liquide aqueux et un effluent liquide hydrocarboné ;

d) optionnellement une étape de fractionnement de tout ou partie de l'effluent hydrocarboné issu de l'étape c), pour obtenir un flux gazeux et au moins deux flux hydrocarbonés qui peuvent être une coupe naphta et une coupe plus lourde ;

e) une étape de recyclage comprenant une phase de récupération d'une fraction de l'effluent hydrocarboné issu de l'étape c) de séparation ou une fraction du et/ou d'au moins un des flux hydrocarboné(s) issu(s) de l'étape d) de fractionnement, vers l'étape a) d'hydrogénation sélective et/ou l'étape b) d'hydrotraitement.

Selon la demande FR20/01.758, la coupe naphta issue de l'étape de fractionnement peut être envoyée, en tout ou partie, soit vers une unité de vapocraquage, soit vers un pool naphta issu de charges pétrolières conventionnelles, soit être recyclée selon l'étape e).

La coupe plus lourde issue de l'étape de fractionnement peut être envoyée, en tout ou partie, soit vers une unité de vapocraquage, soit vers un pool diesel ou kérosène issu de charges pétrolières conventionnelles, soit être recyclée selon l'étape e).

Les demandes de brevet non publiées FR 20/08.108 et FR20/08.106 se basent sur le procédé de FR20/01.758 et décrivent un procédé de traitement d'une huile de pyrolyse de plastiques intégrant une ou deux étapes d'hydrocraquage en lit fixe après l'étape d'hydrotraitement. Ces procédés permettent de minimiser le rendement de la coupe lourde et de maximiser le rendement de la coupe naphta en transformant la coupe lourde au moins en partie en coupe naphta par hydrocraquage, coupe généralement favorisée pour une unité de vapocraquage. Bien que la coupe plus lourde puisse être envoyée vers une unité de vapocraquage, peu de raffineurs favorisent cette option. En effet, la coupe plus lourde a un BMCI élevé et contient par rapport à la coupe naphta plus de composés naphténiques, naphténo-aromatiques et aromatiques menant ainsi à un ratio C/H plus élevé. Ce ratio élevé est une cause de cokage dans le vapocraqueur, nécessitant ainsi des fours de vapocraquage dédiés à cette coupe. De plus, le vapocraquage d'une telle coupe lourde produit moins de produits d'intérêts qui sont notamment l'éthylène et le propylène mais davantage d'essence de pyrolyse.

Du fait de la teneur en impuretés des huiles de pyrolyse, notamment quand elles sont fortement chargées en impuretés, on peut observer une désactivation des catalyseurs de l'unité d'hydrotraitement qui est opérée en lit fixe ce qui diminue la durée de cycle. En effet, la principale contrainte des unités en lit fixe est le fait de devoir arrêter l'unité pour remplacer les catalyseurs. De plus, les huiles de pyrolyse, notamment celles fortement chargées en impuretés, peuvent créer des problèmes de bouchage notamment dans les fours de préchauffe, les échangeurs charge/effluents ou sur les têtes de lits des réacteurs catalytiques.

Il serait donc avantageux de proposer un procédé de traitement d'huiles de pyrolyse ayant des cycles catalytiques de longue durée en permettant le remplacement des catalyseurs sans arrêt de l'unité, tout en produisant une coupe riche en alcanes qui peut être facilement valorisée dans une unité de vapocraquage.

Les unités d'hydroconversion opérées en lit bouillonnant, en lit entraîné ou encore en lit mobile sont capables de traiter ce type de charge grâce à un système d'addition de catalyseur frais et de soutirage de catalyseur usagé sans arrêt de l'unité. L'addition de catalyseur frais et le soutirage de catalyseur usagé sont généralement effectués en continu, en semi-continu ou périodiquement. Ces systèmes qui compensent la désactivation des catalyseurs à cause des impuretés dans les pyrolysats et résolvent les problèmes de bouchage des lits de catalyseurs des réacteurs opérés en lit fixe, permettent aux unités d'hydroconversion d'avoir une longue durée de cycle sans devoir s'arrêter pour remplacer les catalyseurs.

De plus, lorsqu'une telle unité d'hydroconversion est placée en amont d'une unité d'hydrotraitement, la durée de cycle de cette dernière est augmentée grâce aux réactions d'hydrotraitement effectuées en partie au préalable dans l'unité d'hydroconversion.

De même, les réactions d'hydrocraquage effectuées dans l'unité d'hydroconversion permettent de transformer au moins une partie des composés lourds en composés plus légers, ce qui permet premièrement d'alimenter l'unité d'hydrotraitement par une coupe généralement plus facile à traiter et deuxièmement d'obtenir une coupe ayant un BMCI plus faible et donc particulièrement adaptée pour une unité de vapocraquage.

WO2014/001632 divulgue un procédé de traitement d'une charge comprenant une huile de pyrolyse de plastiques et/ou de combustibles solides de récupération.

## RESUME DE L'INVENTION

L'invention concerne un procédé de traitement d'une charge comprenant une huile de pyrolyse de plastiques et/ou de CSR, comprenant :

a) optionnellement, une étape d'hydrogénation sélective mise en œuvre dans une section réactionnelle alimentée au moins par ladite charge et un flux gazeux comprenant de l'hydrogène, en présence d'au moins un catalyseur d'hydrogénation sélective, à une température entre 100 et 280°C, une pression partielle d'hydrogène entre 1,0 et 20,0 MPa abs. et une vitesse volumique horaire entre 0,3 et 10,0 $h^{-1}$, pour obtenir un effluent hydrogéné ;

b) une étape d'hydroconversion mise en œuvre dans une section réactionnelle d'hydroconversion, mettant en œuvre au moins un réacteur à lit bouillonnant, à lit entraîné et/ou à lit mobile, comprenant au moins un catalyseur d'hydroconversion, ladite section réactionnelle d'hydroconversion étant alimentée au moins par ladite charge ou par ledit effluent hydrogéné issu de l'étape a) et un flux gazeux comprenant de l'hydrogène, ladite section réactionnelle d'hydroconversion étant mise en œuvre à une température entre 250 et 450°C, une pression partielle d'hydrogène entre 1,0 et 20,0 MPa abs et une vitesse volumique horaire entre 0,05 et 10,0 $h^{-1}$, pour obtenir un effluent d'hydroconverti ;

c) une étape de séparation, alimentée par l'effluent hydroconverti issu de l'étape b) et une solution aqueuse, ladite étape étant opérée à une température entre 50 et 450°C, pour obtenir au moins un effluent gazeux, un effluent aqueux et un effluent hydrocarboné ;

d) une étape de fractionnement de tout ou partie de l'effluent hydrocarboné issu de l'étape c), pour obtenir au moins un flux gazeux, une coupe hydrocarbonée comprenant des composés ayant un point d'ébullition inférieur ou égal à 385°C et une coupe hydrocarbonée comprenant des composés ayant un point d'ébullition supérieur à 385°C,

e) une étape d'hydrotraitement mise en œuvre dans une section réactionnelle d'hydrotraitement, mettant en œuvre au moins un réacteur à lit fixe ayant n lits catalytiques, n étant un nombre entier supérieur ou égal à 1, comprenant chacun au moins un catalyseur d'hydrotraitement, ladite section réactionnelle d'hydrotraitement étant alimentée par au moins une partie de ladite coupe hydrocarbonée comprenant des composés ayant un point d'ébullition inférieur ou égal à 385°C issue de l'étape d) et un flux gazeux comprenant de l'hydrogène, ladite section réactionnelle d'hydrotraitement étant mise en œuvre à une température entre 250 et 430°C, une pression partielle d'hydrogène entre 1,0 et 20,0 MPa abs. et une vitesse volumique horaire entre 0,1 et 10,0 $h^{-1}$, pour obtenir un effluent hydrotraité ;

f) une étape de séparation, alimentée par l'effluent hydrotraité issu de l'étape e) pour obtenir au moins un effluent gazeux et un effluent hydrocarboné liquide hydrotraité.

Dans la suite du texte, on entend par « huile de pyrolyse » une huile issue de la pyrolyse de plastiques et/ou de CSR, sauf indication contraire.

Un avantage du procédé selon l'invention est de purifier une huile de pyrolyse d'au moins une partie de ses impuretés ce qui permet de l'hydrogéner et ainsi de pouvoir la valoriser en particulier en l'incorporant directement à un pool carburant et/ou encore en la rendant compatible à un traitement dans une unité de vapocraquage afin de pouvoir obtenir en particulier des oléfines légères qui pourront servir de monomères dans la fabrication de polymères.

Un autre avantage de l'invention est de prévenir des risques de bouchage et/ou de corrosion de l'unité de traitement dans laquelle le procédé de l'invention est mis en œuvre, les risques étant exacerbés par la présence, souvent en quantités importantes, de dioléfines, de métaux et de composés halogénés dans l'huile de pyrolyse.

Le procédé de l'invention permet ainsi d'obtenir un effluent hydrocarboné issu d'une huile de pyrolyse débarrassé au moins en partie des impuretés de l'huile de pyrolyse de départ, limitant ainsi les problèmes d'opérabilité, comme les problèmes de corrosion, de cokage ou de désactivation catalytique, que peuvent engendrer ces impuretés, en particulier dans les unités vapocraquage et/ou dans les unités situées en aval des unités de vapocraquage, notamment les unités de polymérisation et d'hydrogénation sélective. L'élimination d'au moins une partie des impuretés des huiles de pyrolyse permettra aussi d'augmenter la gamme des applications des polymères cibles, les incompatibilités d'usages étant réduites.

La présente invention participe au recyclage des plastiques et/ou des CSR, en proposant un procédé de traitement d'une huile issue de la pyrolyse pour la purifier, l'hydroconvertir et l'hydrotraiter. Le fait d'effectuer une étape d'hydroconversion utilisant un système d'addition de catalyseur frais et de soutirage de catalyseur usagé sans arrêt de l'unité en amont d'une étape d'hydrotraitement en lit fixe permet notamment de traiter des huiles de pyrolyse fortement chargées en impuretés.

Le fait d'effectuer une étape d'hydroconversion utilisant un système d'addition de catalyseur frais et de soutirage de catalyseur usagé sans arrêt de l'unité en amont d'une étape d'hydrotraitement en lit fixe permet d'obtenir non seulement des longues durées de cycle pour l'hydroconversion mais permet également de rallonger la durée de cycle de l'étape d'hydrotraitement. De plus, le risque de bouchage du ou des lits catalytiques de l'étape d'hydrotraitement est diminué.

Le fait d'effectuer une étape d'hydroconversion utilisant un système d'addition de catalyseur frais et de soutirage de catalyseur usagé sans arrêt de l'unité en amont d'une étape d'hydrotraitement en lit fixe permet également de transformer au moins une partie des composés lourds en composés plus légers ce qui permet d'obtenir des rendements améliorés en coupe adaptée pour l'unité de vapocraquage et, lorsque cette coupe est envoyée en vapocraquage, en oléfines légères, tout en réduisant en particulier la formation de coke en quantités importantes et/ou les risques de corrosion rencontrés lors d'étape(s) ultérieure(s), par exemple lors d'étape de vapocraquage des huiles de pyrolyse.

La fraction d'huile non convertie par hydroconversion, correspondant à la coupe hydrocarbonée comprenant des composés ayant un point d'ébullition supérieur à 385°C issue de l'étape de fractionnement d), quant à elle est de préférence valorisée en la recyclant dans l'étape d'hydroconversion. De plus, les composés C2 à C4 produits lors de l'hydroconversion peuvent également être envoyés dans le vapocraquage ce qui permet d'améliorer le rendement en oléfines légères (éthylène et propylène).

Selon une variante, le procédé comprend ladite étape a) d'hydrogénation sélective.

Selon une variante, la coupe hydrocarbonée comprenant des composés ayant un point d'ébullition supérieur à 385°C issue de l'étape d) est au moins en partie recyclée à l'étape b).

Selon une variante, le procédé comprend une étape a0) de prétraitement de la charge, ladite étape de prétraitement étant mise en œuvre en amont de l'étape a) d'hydrogénation sélective optionnelle ou en amont de l'étape b) d'hydroconversion et comprend une étape de filtration et/ou une étape de lavage à l'eau et/ou une étape d'adsorption.

Selon une variante, l'effluent hydrocarboné liquide hydrotraité issu de l'étape f), tout ou partie, est envoyée vers une étape h) de vapocraquage réalisée dans au moins un four de pyrolyse à une température comprise entre 700 et 900°C et à une pression comprise entre 0,05 et 0,3 MPa relatif.

Selon une variante, le procédé comprend en outre une étape g) de recyclage dans laquelle une fraction de l'effluent hydrocarboné liquide hydrotraité issu de l'étape f) de séparation, est envoyée vers l'étape a) d'hydrogénation sélective optionnelle et/ou l'étape b) d'hydroconversion et/ou l'étape e) d'hydrotraitement et/ou l'étape e') d'hydrocraquage.

Selon une variante, l'étape de séparation f) comprend un fractionnement permettant d'obtenir, outre un flux gazeux, une coupe naphta comprenant des composés ayant un point d'ébullition inférieur ou égal à 175°C, et une coupe diesel comprenant des composés ayant un point d'ébullition supérieur à 175°C et inférieur à 385°C.

Selon une variante, le procédé comprend en outre une étape e') d'hydrocraquage mise en œuvre dans une section réactionnelle d'hydrocraquage, mettant en œuvre au moins un lit fixe ayant n lits catalytiques, n étant un nombre entier supérieur ou égal à 1, comprenant chacun au moins un catalyseur d'hydrocraquage, ladite section réactionnelle d'hydrocraquage étant alimentée au moins par ledit effluent hydrotraité issu de l'étape e) et/ou par la coupe diesel comprenant des composés ayant un point d'ébullition supérieur à 175°C et inférieur à 385°C issue de l'étape f) et un flux gazeux comprenant de l'hydrogène, ladite section réactionnelle d'hydrocraquage étant mise en œuvre à une température entre 250 et 450°C, une pression partielle d'hydrogène entre 1,5 et 20,0 MPa abs. et une vitesse volumique horaire entre 0,1 et 10,0 $h^{-1}$, pour obtenir un effluent hydrocraqué qui est envoyé dans l'étape f) de séparation.

Selon une variante, l'étape séparation f) comprend en outre un fractionnement de la coupe naphta comprenant des composés ayant un point d'ébullition inférieur ou égal à 175°C en une coupe naphta légère comprenant des composés ayant un point d'ébullition inférieure à 80°C et une coupe naphta lourde comprenant des composés ayant un point d'ébullition entre 80 et 175°C.

Selon cette variante, au moins une partie de ladite coupe naphta lourde est envoyée vers un complexe aromatique comportant au moins une étape de reformage du naphta et/ou dans lequel au moins une partie de la coupe naphta légère est envoyée dans l'étape h) de vapocraquage.

Selon une variante, ledit catalyseur d'hydrogénation sélective de l'étape a) comprend un support choisi parmi l'alumine, la silice, les silices-alumines, la magnésie, les argiles et leurs mélanges et une fonction hydro-déshydrogénante comprenant soit au moins un élément du groupe VIII et au moins un élément du groupe VIB, soit au moins un élément du groupe VIII.

Selon une variante, lorsque l'étape b) est mise en œuvre en lit bouillonnant ou en lit mobile, ledit catalyseur d'hydroconversion de l'étape b) comprend un catalyseur supporté comprenant un métal du groupe VIII choisi dans le groupe formé par le Ni, Pd, Pt, Co, Rh et/ou Ru, optionnellement un métal du groupe VIB choisi dans le groupe Mo et/ou W, sur un support minéral amorphe choisi dans le groupe formé par l'alumine, la silice, les silices-alumines, la magnésie, les argiles et les mélanges d'au moins deux de ces minéraux, et lorsque l'étape b) est mis en œuvre en lit entrainé, ledit catalyseur d'hydroconversion de l'étape b) comprend un catalyseur dispersé contenant au moins un élément choisi dans le groupe formé par Mo, Fe, Ni, W, Co, V, Ru.

Selon une variante, ledit catalyseur d'hydrotraitement de l'étape e) comprend un support choisi dans le groupe constitué par l'alumine, la silice, les silices-alumines, la magnésie, les argiles et leurs mélanges, et une fonction hydro-déshydrogénante comprenant au moins un élément du groupe VIII et/ou au moins un élément du groupe VIB.

Selon une variante, ledit catalyseur d'hydrocraquage de l'étape e') comprend un support choisi parmi les alumines halogénées, les combinaisons d'oxydes de bore et d'aluminium, les silice-alumines amorphes et les zéolithes et une fonction hydro-déshydrogénante comprenant au moins un métal du groupe VIB choisi parmi le chrome, le molybdène et le tungstène, seul ou en mélange, et/ou au moins un métal du groupe VIII choisi parmi le fer, le cobalt, le nickel, le ruthénium, le rhodium, le palladium et le platine.

Selon une variante, la charge a les propriétés suivantes :

-   une teneur en aromatiques comprise entre 0 et 90 % poids,
-   une teneur en halogénés comprise entre 2 et 5000 ppm poids,
-   une teneur en éléments métalliques comprise entre 10 et 10000 ppm poids,
-   dont une teneur en élément fer comprise entre 0 et 100 ppm poids,
-   une teneur en élément silicium comprise entre 0 et 1000 ppm poids.

L'invention concerne également le produit susceptible d'être obtenu par le procédé de traitement selon l'invention.

Selon une variante, le produit comporte par rapport au poids total du produit :

-   une teneur totale en éléments métalliques inférieure ou égale à 5,0 ppm poids,
-   dont une teneur en élément fer inférieure ou égale à 100 ppb poids,
-   une teneur en élément silicium inférieure ou égale à 1,0 ppm poids,
-   une teneur en soufre inférieure ou égale à 500 ppm poids,
-   une teneur en azote inférieure ou égale à 100 ppm poids,
-   une teneur en élément chlore inférieure ou égale à 10 ppm poids.

Selon la présente invention, les pressions sont des pressions absolues, encore notées abs., et sont données en MPa absolu (ou MPa abs.), sauf indication contraire.

Selon la présente invention, les expressions « compris entre ... et ... » et « entre .... et ... » sont équivalentes et signifient que les valeurs limites de l'intervalle sont incluses dans la gamme de valeurs décrite. Si tel n'était pas le cas et que les valeurs limites n'étaient pas incluses dans la gamme décrite, une telle précision sera apportée par la présente invention.

Dans le sens de la présente invention, les différentes plages de paramètres pour une étape donnée telles que les plages de pression et les plages de température peuvent être utilisées seules ou en combinaison. Par exemple, dans le sens de la présente invention, une plage de valeurs préférées de pression peut être combinée avec une plage de valeurs de température plus préférées.

Dans la suite, des modes de réalisation particuliers et/ou préférés de l'invention peuvent être décrits. Ils pourront être mis en œuvre séparément ou combinés entre eux, sans limitation de combinaison lorsque c'est techniquement réalisable.

Dans la suite, les groupes d'éléments chimiques sont donnés selon la classification CAS (CRC Handbook of Chemistry and Physics, éditeur CRC press, rédacteur en chef D.R. Lide, 81ème édition, 2000-2001). Par exemple, le groupe VIII selon la classification CAS correspond aux métaux des colonnes 8, 9 et 10 selon la nouvelle classification IUPAC.

La teneur en métaux est mesurée par fluorescence X.

## DESCRIPTION DETAILLEE

### La charge

Selon l'invention, une « huile de pyrolyse de plastiques ou huile de pyrolyse de CSR » est une huile, avantageusement sous forme liquide à température ambiante, issue de la pyrolyse de plastiques, de préférence de déchets plastiques provenant notamment de filières de collecte et de tri, ou issue de la pyrolyse de CSR. Elle comprend en particulier un mélange de composés hydrocarbonés, notamment des paraffines, des oléfines, des naphtènes et des aromatiques. Au moins 80% poids de ces composés hydrocarbonés ont de préférence un point d'ébullition inférieur à 700°C, et de manière préférée inférieur à 550°C. En particulier, selon l'origine de l'huile de pyrolyse, celle-ci peut comprendre jusqu'à 70% poids en paraffines, jusqu'à 90 % poids en oléfines et jusqu'à 90 % poids en aromatiques, étant entendu que la somme des paraffines, des oléfines et des aromatiques est 100 % poids des composées hydrocarbonés.

La densité de l'huile de pyrolyse, mesurée à 15°C selon la méthode ASTM D4052, est généralement comprise entre 0,75 et 0,99 g/cm$^3$, de préférence comprise entre 0,75 et 0,95 g/cm$^3$.

L'huile de pyrolyse peut comprendre, et le plus souvent comprend, en outre des impuretés comme des métaux, notamment du fer, du silicium, des composés halogénés, notamment des composés chlorés. Ces impuretés peuvent être présentes dans l'huile de pyrolyse à des teneurs élevées, par exemple jusqu'à 500 ppm poids ou encore 1000 ppm poids voire 5000 ppm poids d'éléments halogène apportés par des composés halogénés, et jusqu'à 2500 ppm poids, voire 10000 ppm poids d'éléments métalliques ou semi-métalliques. Les métaux alcalins, les alcalino terreux, les métaux de transition, les métaux pauvres et les métalloïdes peuvent être assimilés aux contaminants de nature métallique, appelés métaux ou éléments métalliques ou semi métalliques. L'huile de pyrolyse peut comprendre jusqu'à 200 ppm poids ou encore 1000 ppm poids de silicium, et jusqu'à 15 ppm poids ou encore 100 ppm poids de fer. L'huile de pyrolyse peut également comprendre d'autres impuretés comme des hétéroéléments apportés notamment par des composés soufrés, des composés oxygénés et/ou des composés azotés, à des teneurs généralement inférieures à 20000 ppm poids d'hétéroéléments et de préférence inférieures à 10000 ppm poids d'hétéroéléments.

Le procédé selon l'invention est particulièrement bien adapté pour traiter une huile de pyrolyse chargée en impuretés. On entend par là, une charge ayant les propriétés suivantes :

- une teneur en aromatiques comprise entre 0 et 90 % poids, souvent comprise entre 20 et 90 % poids, et pouvant être comprise entre 50 et 90 % poids ;
- une teneur en halogénés comprise entre 2 et 5000 ppm poids, souvent comprise entre 200 et 5000 ppm poids, et pouvant être comprise entre 500 et 5000 ppm poids ;
- une teneur en éléments métalliques comprise entre 10 et 10000 ppm poids, souvent comprise entre 2000 et 10000 ppm poids, et pouvant être comprise entre 2250 et 5000 ppm poids ;
- dont une teneur en élément fer comprise entre 0 et 100 ppm poids, souvent comprise entre 10 et 100 ppm poids, et pouvant être comprise entre 15 et 100 ppm poids ;
- une teneur en élément silicium comprise entre 0 et 1000 ppm poids, souvent comprise entre 100 et 1000 ppm poids, et pouvant être comprise entre 200 et 1000 ppm poids.

Le procédé selon l'invention est particulièrement bien adapté pour traiter une huile de pyrolyse fortement chargée en impuretés. On entend par là, une charge ayant les propriétés suivantes :

- une teneur en aromatiques comprise entre 50 et 90 % poids ;
- une teneur en halogénés comprise entre 500 et 5000 ppm poids ;
- une teneur en éléments métalliques comprise entre 2250 et 5000 ppm poids ;
- dont une teneur en élément fer comprise entre 15 et 100 ppm poids ;
- une teneur en élément silicium comprise 200 et 1000 ppm poids.

La charge du procédé selon l'invention comprend au moins une huile de pyrolyse de plastiques et/ou de CSR. Ladite charge peut être constituée uniquement d'huile(s) de pyrolyse de plastiques ou uniquement d'huile(s) de pyrolyse de CSR ou uniquement d'un mélange d'huile(s) de pyrolyse de plastiques et de CSR. De préférence, ladite charge comprend au moins 50% poids, de manière préférée entre 50 et 100% poids, et de manière particulièrement préférée entre 75 et 100% poids d'huile de pyrolyse de plastiques et/ou de CSR.

La charge du procédé selon l'invention peut comprendre en outre une charge pétrolière conventionnelle et/ou une charge issue de la conversion de ressource lignocellulosique qui est alors co-traitée avec l'huile de pyrolyse de plastiques et/ou de CSR.

L'huile de pyrolyse de plastiques et/ou de CSR peut être issue d'un traitement de pyrolyse thermique, catalytique ou encore être préparée par hydropyrolyse (pyrolyse en présence d'un catalyseur et d'hydrogène).

**Prétraitement (optionnel)**

Ladite charge comprenant une huile de pyrolyse peut avantageusement être prétraitée dans une étape optionnelle de prétraitement a0), préalablement à l'étape a) d'hydrogénation sélective optionnelle ou à l'étape b) d'hydroconversion lorsque l'étape a) n'est pas présente, pour obtenir une charge prétraitée qui alimente l'étape a) ou l'étape b).

Cette étape optionnelle de prétraitement a0) permet de diminuer la quantité de contaminants, en particulier la quantité de silicium et de métaux, éventuellement présents dans la charge comprenant l'huile de pyrolyse. Ainsi, une étape optionnelle a0) de prétraitement de la charge comprenant une huile de pyrolyse peut être réalisée en particulier lorsque ladite charge comprend plus de 50 ppm poids, notamment plus de 100 ppm poids, plus particulièrement plus de 200 ppm poids d'éléments métalliques.

Ladite étape optionnelle de prétraitement a0) peut être mise en œuvre par n'importe quelle méthode connue par l'homme du métier permettant de diminuer la quantité de contaminants. Elle peut notamment comprendre une étape de filtration et/ou une étape de lavage à l'eau et/ou une étape d'adsorption.

Selon une variante, ladite étape optionnelle de prétraitement a0) est mise en œuvre dans une section d'adsorption opérée en présence d'au moins un adsorbant. Ladite étape optionnelle de prétraitement a0) est mise en œuvre à une température entre 0 et 150°C, de préférence entre 5 et 100°C, et à une pression entre 0,15 et 10,0 MPa abs, de préférence entre 0,2 et 1,0 MPa abs. La section d'adsorption est opérée avantageusement en présence d'au moins un adsorbant, de préférence de type alumine, ayant une surface spécifique supérieure ou égale à 100 $m^2$/g, de préférence supérieure ou égale à 200 $m^2$/g. La surface spécifique dudit adsorbant est avantageusement inférieure ou égale à 600 $m^2$/g, en particulier inférieure ou égale à 400 $m^2$/g. La surface spécifique de l'adsorbant est une surface mesurée par la méthode BET, c'est-à-dire la surface spécifique déterminée par adsorption d'azote conformément à la norme ASTM D 3663 établie à partir de la méthode BRUNAUER-EMMETT-TELLER décrite dans le périodique 'The Journal of the American Chemical Society", 6Q, 309 (1938).

Avantageusement, ledit adsorbant comprend moins de 1% poids d'éléments métalliques, de préférence est exempt d'éléments métalliques. Par éléments métalliques de l'adsorbant, il faut entendre les éléments des groupes 6 à 10 du tableau périodique des éléments (nouvelle classification IUPAC).

Ladite section d'adsorption de l'étape optionnelle a0) comprend au moins une colonne d'adsorption, de préférence comprend au moins deux colonnes d'adsorption, préférentiellement entre deux et quatre colonnes d'adsorption, contenant ledit adsorbant. Lorsque la section d'adsorption comprend deux colonnes d'adsorption, un mode de fonctionnement peut être un fonctionnement appelé « en swing », selon le terme anglo-saxon consacré, dans lequel l'une des colonnes est en ligne, c'est-à-dire en fonctionnement, tandis que l'autre colonne est en réserve. Lorsque l'absorbant de la colonne en ligne est usé, cette colonne est isolée tandis que la colonne en réserve est mise en ligne, c'est-à-dire en fonctionnement. L'absorbant usé peut être ensuite régénéré *in situ* et/ou remplacé par de l'absorbant frais pour que la colonne le contenant puisse à nouveau être remise en ligne une fois que l'autre colonne aura été isolée.

Un autre mode de fonctionnement est d'avoir au moins deux colonnes fonctionnant en série. Lorsque l'absorbant de la colonne placée en tête est usé, cette première colonne est isolée et l'absorbant usée est soit régénéré *in situ* ou remplacé par de l'absorbant frais. La colonne est ensuite remise en ligne en dernière position et ainsi de suite. Ce fonctionnement est appelé mode permutable, ou selon le terme anglais « PRS » pour Permutable Reactor System ou encore « lead and lag » selon le terme anglo-saxon consacré. L'association d'au moins deux colonnes d'adsorption permet de palier à l'empoisonnement et/ou au colmatage possible et éventuellement rapide de l'adsorbant sous l'action conjointe des contaminants métalliques, des dioléfines, des gommes issues des dioléfines et des insolubles éventuellement présents dans l'huile de pyrolyse à traiter. La présence d'au moins deux colonnes d'adsorption facilite en effet le remplacement et/ou la régénération de l'adsorbant, avantageusement sans arrêt de l'unité de prétraitement, voire du procédé, permettant ainsi de diminuer les risques de colmatage et donc d'éviter l'arrêt de l'unité dû au colmatage, de maitriser les coûts et de limiter la consommation d'adsorbant.

Ladite étape optionnelle a0) de prétraitement peut également être éventuellement alimentée par au moins une fraction d'un flux de recycle, avantageusement issu de l'étape g) du procédé, en mélange ou séparément de la charge comprenant une huile de pyrolyse.

Ladite étape optionnelle a0) de prétraitement permet ainsi d'obtenir une charge prétraitée qui alimente ensuite l'étape a) d'hydrogénation sélective lorsqu'elle est présente, ou l'étape b) d'hydroconversion.

**Etape a) d'hydrogénation sélective (optionnelle)**

Selon l'invention, le procédé peut comprendre une étape a) d'hydrogénation sélective de la charge comprenant une huile de pyrolyse réalisée en présence d'hydrogène, dans des conditions de pression en hydrogène et de température permettant de maintenir ladite charge en phase liquide et avec une quantité d'hydrogène soluble juste nécessaire à une hydrogénation sélective des dioléfines présentes dans l'huile de pyrolyse. L'hydrogénation sélective des dioléfines en phase liquide permet ainsi d'éviter ou au moins de limiter la formation de « gommes », c'est-à-dire la polymérisation des dioléfines et donc la formation d'oligomères et polymères, pouvant boucher la section réactionnelle de l'étape e) d'hydrotraitement. Ladite étape a) d'hydrogénation sélective permet d'obtenir un effluent hydrogéné sélectivement, c'est-à-dire un effluent à teneur réduite en oléfines, en particulier en dioléfines.

Selon l'invention, ladite étape a) d'hydrogénation sélective est mise en œuvre dans une section réactionnelle alimentée au moins par ladite charge comprenant une huile de pyrolyse, ou par la charge prétraitée issue de l'éventuelle étape a0) de prétraitement, et un flux gazeux comprenant de l'hydrogène ($H_2$). Eventuellement, la section réactionnelle de ladite étape a) peut également être alimentée en outre par au moins une fraction d'un flux de recycle, avantageusement issu de l'étape g) optionnelle, soit en mélange avec ladite charge, éventuellement prétraitée, soit séparément de la charge, éventuellement prétraitée, avantageusement directement en entrée d'au moins un des réacteurs de la section réactionnelle de l'étape a). L'introduction d'au moins une fraction dudit flux de recycle dans la section réactionnelle de l'étape a) d'hydrogénation sélective permet avantageusement de diluer les impuretés de la charge, éventuellement prétraitée, et de contrôler la température notamment dans ladite section réactionnelle.

Ladite section réactionnelle met en œuvre une hydrogénation sélective, de préférence en lit fixe, en présence d'au moins un catalyseur d'hydrogénation sélective, avantageusement à une température entre 100 et 280°C, de préférence entre 120 et 260°C, de manière préférée entre 130 et 250°C, une pression partielle d'hydrogène entre 1,0 et 20,0 MPa abs, de manière préférée entre 5,0 et 15,0 MPa abs et à une vitesse volumique horaire (VVH) entre 0,3 et 10,0 h$^{-1}$, de manière préférée entre 0,5 et 5,0 h$^{-1}$. La vitesse volumique horaire (VVH) est définie ici comme le ratio entre le débit volumique horaire de la charge comprenant l'huile de pyrolyse, éventuellement prétraitée, par le volume de catalyseur(s). La quantité du flux gazeux comprenant de l'hydrogène (H$_2$), alimentant ladite section réactionnelle de l'étape a), est avantageusement telle que la couverture en hydrogène est comprise entre 1 et 200 Nm$^3$ d'hydrogène par m$^3$ de charge (Nm$^3$/m$^3$), de préférence entre 1 et 50 Nm$^3$ d'hydrogène par m$^3$ de charge (Nm$^3$/m$^3$), de manière préférée entre 5 et 20 Nm$^3$ d'hydrogène par m$^3$ de charge (Nm$^3$/m$^3$). La couverture en hydrogène est définie comme le rapport du débit volumique d'hydrogène pris dans les conditions normales de température et pression par rapport au débit volumique de charge « fraîche », c'est-à-dire de la charge à traiter, éventuellement prétraitée, sans tenir compte de l'éventuelle fraction recyclée, à 15°C (en normaux m$^3$, noté Nm$^3$, de H$_2$ par m$^3$ de charge). Le flux gazeux comprenant de l'hydrogène, qui alimente la section réactionnelle de l'étape a), peut être constitué d'un appoint en hydrogène et/ou d'hydrogène recyclé issu en particulier de l'étape c) de séparation.

L'étape a) d'hydrogénation sélective est de préférence effectuée en lit fixe. Elle peut également être effectuée en lit bouillonnant ou en lit mobile.

Avantageusement, la section réactionnelle de ladite étape a) comprend entre 1 et 5 réacteurs. Selon un mode de réalisation particulier de l'invention, la section réactionnelle comprend entre 2 et 5 réacteurs, qui fonctionnent en mode permutable, appelé selon le terme anglais « PRS » pour Permutable Reactor System ou encore « lead and lag ». L'association d'au moins deux réacteurs en mode PRS permet d'isoler un réacteur, de décharger le catalyseur usé, de recharger le réacteur en catalyseur frais et remettre en service ledit réacteur sans arrêt du procédé. La technologie PRS est décrite, en particulier, dans le brevet FR2681871.

Avantageusement, des internes de réacteurs, par exemple de type plateaux filtrants, peuvent être utilisés pour prévenir le bouchage du(des) réacteur(s). Un exemple de plateau filtrant est décrit dans le brevet FR3051375.

Avantageusement, ledit catalyseur d'hydrogénation sélective comprend un support, de préférence minéral, et une fonction hydro-déshydrogénante.

Selon une variante, la fonction hydro-déshydrogénante comprend en particulier au moins un élément du groupe VIII, de préférence choisi parmi le nickel et le cobalt, et au moins un élément du groupe VIB, de préférence choisi parmi le molybdène et le tungstène. Selon cette variante, la teneur totale en oxydes des éléments métalliques des groupes VIB et VIII est de préférence comprise entre 1% et 40% en poids, préférentiellement de 5% à 30% en poids par rapport au poids total du catalyseur. Le rapport pondéral exprimé en oxyde métallique entre le métal (ou les métaux) du groupe VIB par rapport au métal (ou aux métaux) du groupe VIII est de préférence compris entre 1 et 20, et de manière préférée entre 2 et 10. Selon cette variante, la section réactionnelle de ladite étape a) comprend par exemple un catalyseur d'hydrogénation sélective comprenant entre 0,5% et 12% en poids de nickel, de préférence entre 1% et 10% en poids de nickel (exprimé en oxyde de nickel NiO par rapport au poids dudit catalyseur), et entre 1% et 30% en poids de molybdène, de préférence entre 3% et 20% en poids de molybdène (exprimé en oxyde de molybdène MoOs par rapport au poids dudit catalyseur) sur un support de préférence minéral, de préférence sur un support d'alumine.

Selon une autre variante, la fonction hydro-déshydrogénante comprend, et est de préférence constituée d'au moins un élément du groupe VIII, de préférence du nickel. Selon cette variante, la teneur en oxydes de nickel est de préférence comprise entre 1 et 50 % en poids, de préférence entre 10% et 30% en poids par rapport au poids dudit catalyseur. Ce type de catalyseur est de préférence utilisé sous sa forme réduite, sur un support de préférence minéral, de préférence sur un support d'alumine.

Le support dudit au moins catalyseur d'hydrogénation sélective est de préférence choisi parmi l'alumine, la silice, les silices-alumines, la magnésie, les argiles et leurs mélanges. Ledit support peut renfermer des composés dopants, notamment des oxydes choisis parmi l'oxyde de bore, en particulier le trioxyde de bore, la zircone, la cérine, l'oxyde de titane, l'anhydride phosphorique et un mélange de ces oxydes. De préférence, ledit au moins catalyseur d'hydrogénation sélective comprend un support d'alumine, éventuellement dopé avec du phosphore et éventuellement du bore. Lorsque l'anhydride phosphorique P$_2$O$_5$ est présent, sa concentration est inférieure à 10% en poids par rapport au poids de l'alumine et avantageusement d'au moins 0,001 % poids par rapport au poids total de l'alumine. Lorsque le trioxyde de bore B$_2$O$_5$ est présent, sa concentration est inférieure à 10% en poids par rapport au poids de l'alumine et avantageusement d'au moins 0,001 % par rapport au poids total de l'alumine. L'alumine utilisée peut être par exemple une alumine $\gamma$ (gamma) ou $\eta$ (éta).

Ledit catalyseur d'hydrogénation sélective est par exemple sous forme d'extrudés.

De manière très préférée, afin d'hydrogéner les dioléfines le plus sélectivement possible, l'étape a) peut mettre en œuvre en plus des catalyseurs d'hydrogénation sélective décrits ci-dessus en outre au moins un catalyseur d'hydrogénation sélective utilisé dans l'étape a) comprenant moins de 1% en poids de nickel et au moins 0,1% poids de nickel, de préférence 0,5% poids de nickel, exprimé en oxyde de nickel NiO par rapport au poids dudit catalyseur, et moins de 5% en poids de molybdène et au moins 0,1 % poids de molybdène, de préférence 0,5% poids de molybdène, exprimé en oxyde de molybdène MoOs par rapport au poids dudit catalyseur, sur un support d'alumine. Ce catalyseur peu chargé en métaux est de préférence mis en amont des catalyseurs d'hydrogénation sélective décrits ci-dessus.

Eventuellement, la charge qui comprend une huile de pyrolyse, éventuellement prétraitée, et/ou éventuellement mélangée préalablement avec au moins une fraction d'un flux de recycle, avantageusement issu de l'étape g) optionnelle, peut être mélangée avec le flux gazeux comprenant de l'hydrogène préalablement à son introduction dans la section réactionnelle.

Ladite charge, éventuellement prétraitée, et/ou éventuellement mélangée avec au moins une fraction du flux de recycle, avantageusement issu de l'étape g) optionnelle, et/ou éventuellement en mélange avec le flux gazeux, peut également être chauffée avant son introduction dans la section réactionnelle de l'étape a), par exemple par échange de chaleur notamment avec l'effluent d'hydroconverti de l'étape b), pour atteindre une température proche de la température mise en œuvre dans la section réactionnelle qu'elle alimente.

La teneur en impuretés, en particulier en dioléfines, de l'effluent hydrogéné obtenu à l'issue de l'étape a) est réduite par rapport à celle des mêmes impuretés, en particulier des dioléfines, comprises dans la charge du procédé. L'étape a) d'hydrogénation sélective permet généralement de convertir au moins 90% et de préférence au moins 99% des dioléfines contenues dans la charge initiale. L'étape a) permet également l'élimination, au moins en partie, d'autres contaminants, comme par exemple le silicium. L'effluent hydrogéné, obtenu à l'issue de l'étape a) d'hydrogénation sélective, est envoyé, de préférence directement, vers l'étape b) d'hydroconversion.

**Etape b) d'hydroconversion**

Selon l'invention, le procédé de traitement comprend une étape b) d'hydroconversion mise en œuvre dans une section réactionnelle d'hydroconversion, mettant en œuvre au moins un réacteur à lit bouillonnant, à lit en lit entraîné et/ou encore à lit mobile, comprenant au moins un catalyseur d'hydroconversion, ladite section réactionnelle d'hydroconversion étant alimentée au moins par ladite charge ou par ledit effluent hydrogéné issu de l'étape a), éventuellement en mélange avec au moins une fraction d'un flux de recycle, avantageusement issu de l'étape g) optionnelle et un flux gazeux comprenant de l'hydrogène, pour obtenir un effluent d'hydroconverti.

Avantageusement, l'étape b) met en œuvre les réactions d'hydroconversion bien connues de l'homme du métier, et plus particulièrement des réactions d'hydrotraitement telles que l'hydrogénation des oléfines, des aromatiques, des composés halogénés, l'hydrodémétallation, l'hydrodésulfuration, l'hydrodéazotation, etc. et des réactions d'hydrocraquage (HCK) qui conduisent à l'ouverture de cycle naphténique ou le fractionnement de paraffines en plusieurs fragments de plus faible poids moléculaire, des réactions de craquage thermique et de polycondensation (formation de coke) bien que ces dernières ne soient pas désirées.

Ladite section réactionnelle d'hydroconversion de l'étape b) peut également être alimentée par au moins une fraction du flux de recycle, avantageusement issue de l'étape g) optionnelle. La(les)dite(s) fraction(s) dudit flux de recycle ou la totalité du flux de recycle peu(ven)t être introduite(s) dans ladite section réactionnelle d'hydroconversion en mélange avec la charge ou l'effluent hydrogéné issu de l'étape a), ou séparément. L'introduction d'au moins une fraction dudit flux de recycle permet avantageusement de diluer les impuretés encore présentes dans l'effluent hydrogéné et de contrôler la température, en particulier de limiter l'augmentation de température, dans le ou les lit(s) catalytique(s) de la section réactionnelle d'hydroconversion qui met en œuvre des réactions fortement exothermiques.

Eventuellement, l'étape b) peut mettre en œuvre une section de chauffe située en amont de la section réactionnelle d'hydroconversion et dans laquelle la charge ou l'effluent hydrogéné issu de l'étape a) est chauffé pour atteindre une température adaptée pour l'hydroconversion, c'est-à-dire une température comprise entre 250 et 450°C. Ladite éventuelle section de chauffe peut ainsi comprendre un ou plusieurs échangeurs, permettant de préférence un échange de chaleur entre la charge ou l'effluent hydrogéné et l'effluent d'hydroconverti, et/ou un four de préchauffe.

Avantageusement, ladite section réactionnelle d'hydroconversion est mise en œuvre à une pression équivalente à celle utilisée dans la section réactionnelle de l'étape a) d'hydrogénation sélective lorsqu'elle est présente, mais à une plus haute température que celle de la section réactionnelle de l'étape a) d'hydrogénation sélective. Ainsi, ladite section réactionnelle d'hydroconversion, et ceci peu importe si on utilise une section réactionnelle à lit bouillonnant, à lit entraîné ou/ou à lit mobile, est avantageusement mise en œuvre à une température d'hydroconversion entre 250 et 450°C, de préférence entre 350 et 420°C, à une pression partielle d'hydrogène entre 1,0 et 20,0 MPa abs., plus préférentiellement entre 5,0 et 15,0 MPa abs, et à une vitesse volumique horaire (VVH) entre 0,05 et 10,0 $h^{-1}$, de préférence entre 0,1 et 5,0 $h^{-1}$. Selon l'invention, la « température d'hydroconversion » correspond à une température moyenne dans la section réactionnelle d'hydroconversion de l'étape b). La température d'hydroconversion est avantageusement déterminée en fonction des systèmes catalytiques, des équipements, de la configuration de ceux-ci, par l'homme du métier. Par exemple, la température d'hydroconversion en lit bouillonnant est déterminée en faisant la moyenne arithmétique des mesures de températures dans le lit catalytique. La vitesse volumique horaire (VVH) est définie ici comme le ratio entre le débit volumique horaire de l'effluent hydrogéné issu de l'étape a) par volume de catalyseur(s). La couverture en hydrogène dans l'étape b) est avantageusement comprise entre 50 et 1000 Nm$^3$ d'hydrogène par m$^3$ de charge fraîche qui alimente l'étape a), et de préférence entre 60 et 500 Nm$^3$ d'hydrogène par m$^3$ de charge fraîche qui alimente l'étape a), de manière préférée entre 100 et 300 Nm$^3$ d'hydrogène par m$^3$ de charge fraîche qui alimente l'étape a). La couverture en hydrogène est définie ici comme le rapport du débit volumique d'hydrogène pris dans les conditions normales de température et pression par rapport au débit volumique de charge fraîche qui alimente l'étape a), c'est-à-dire de charge comprenant une huile de pyrolyse, ou par la charge éventuellement prétraitée, qui alimente l'étape a) (en normaux m$^3$, noté Nm$^3$, de H$_2$ par m$^3$ de charge fraîche). L'hydrogène peut être constitué d'un appoint et/ou d'hydrogène recyclé issu en particulier de l'étape c) de séparation.

Une caractéristique importante du procédé selon l'invention est le fait que l'étape d'hydroconversion est effectuée dans une section réactionnelle permettant l'addition de catalyseur frais et de soutirage de catalyseur usagé sans arrêt de l'unité. De tels systèmes sont des unités d'hydroconversion opérées en lit bouillonnant, en lit entraîné et/ou encore en lit mobile. L'addition de catalyseur frais et de soutirage de catalyseur usagé peuvent ainsi être effectués en continu, en semi-continu ou périodiquement.

Etape b) d'hydroconversion en lit bouillonnant

Ainsi, selon une première variante, l'étape b) d'hydroconversion est mise en œuvre dans une section réactionnelle d'hydroconversion mettant en œuvre au moins un réacteur à lit bouillonnant.

Le fonctionnement du réacteur à lit bouillonnant, y compris le recyclage des liquides du réacteur vers le haut au travers du lit de catalyseur agité est généralement bien connu. On fait passer un mélange de charge et d'hydrogène de bas en haut sur un lit de particules catalytiques à un débit tel que les particules sont soumises à un mouvement aléatoire forcé tandis que le liquide et le gaz traversent le lit de bas en haut. Le mouvement du lit catalytique est contrôlé par un flux de liquide de recyclage de telle manière que, en régime stationnaire, la masse du catalyseur ne s'élève pas au-dessus d'un niveau définissable dans le réacteur. Des vapeurs et le liquide en train d'être hydrogéné passent à travers le niveau supérieur du lit de particules catalytiques pour atteindre une zone sensiblement exempte de catalyseur, puis ils sont évacués de la partie supérieure du réacteur. Une fraction des liquides du réacteur est en permanence recyclée dans le réacteur. Les technologies à lits bouillonnants utilisent des catalyseurs supportés, généralement sous forme d'extrudés ou de billes dont le diamètre est généralement de l'ordre de 1mm ou inférieur à 1mm. Les catalyseurs restent à l'intérieur des réacteurs et ne sont pas évacués avec les produits. L'activité catalytique peut être maintenue constante grâce au remplacement en ligne du catalyseur. Il n'est donc pas nécessaire d'arrêter l'unité pour changer le catalyseur usagé, ni d'augmenter les températures de réaction le long du cycle pour compenser la désactivation. De plus, le fait de travailler à des conditions opératoires constantes permet d'obtenir des rendements et des qualités de produits constants le long du cycle. Aussi, du fait que le catalyseur est maintenu en agitation par un recyclage important de liquide, la perte de charge sur le réacteur reste faible et constante, et les exothermes de réaction sont rapidement moyennés sur le lit catalytique.

Le catalyseur usagé est en partie remplacé par du catalyseur frais par soutirage en bas du réacteur et introduction, soit en haut du réacteur soit en bas du réacteur, de catalyseur frais ou neuf à intervalle de temps régulier, c'est-à-dire par exemple par bouffée ou de façon quasi continue. On peut par exemple introduire du catalyseur frais tous les jours. Le taux de remplacement du catalyseur usé par du catalyseur frais peut être par exemple d'environ 0,01 kilogramme à environ 10 kilogrammes par mètre cube de charge. Ce soutirage et ce remplacement sont effectués à l'aide de dispositifs permettant le fonctionnement continu de cette étape d'hydroconversion. L'unité comporte habituellement une pompe de recirculation interne permettant le maintien du catalyseur en lit bouillonnant par recyclage continu d'au moins une partie du liquide soutiré en tête du réacteur et réinjecté en bas du réacteur. Il est également possible d'envoyer le catalyseur usé soutiré du réacteur dans une zone de régénération dans laquelle on élimine le carbone et le soufre qu'il renferme, puis de renvoyer ce catalyseur régénéré dans l'étape d'hydroconversion. Il est également possible d'envoyer le catalyseur régénéré dans une zone de réjuvénation dans laquelle on effectue un traitement visant à améliorer l'activité du catalyseur (présulfuration, additivation...), puis de renvoyer ce catalyseur réjuvéné dans l'étape d'hydroconversion.

Les catalyseurs utilisés en lit bouillonnant sont largement commercialisés. Ce sont des catalyseurs granulaires dont la taille n'atteint jamais celles des catalyseurs utilisés en lit entraîné. Le catalyseur est le plus souvent sous forme d'extrudés ou de billes. Typiquement, ils contiennent au moins un élément hydro-déshydrogénant déposé sur un support amorphe. Généralement, le catalyseur supporté comprend un métal du groupe VIII choisi dans le groupe formé par le Ni, Pd, Pt, Co, Rh et/ou Ru, optionnellement un métal du groupe VIB choisi dans le groupe Mo et/ou W, sur un support minéral amorphe choisi dans le groupe formé par l'alumine, la silice, les silices-alumines, la magnésie, les argiles et les mélanges d'au moins deux de ces minéraux. Les catalyseurs CoMo/alumine et NiMo/alumine sont les plus courants.

La teneur totale en oxydes des éléments métalliques des groupes VIB et VIII est de préférence entre 0,1% et 40% en poids, préférentiellement de 5% à 35% en poids, par rapport au poids total du catalyseur. Le rapport pondéral exprimé en oxyde métallique entre le métal (ou les métaux) du groupe VIB par rapport au métal (ou aux métaux) du groupe VIII est de préférence compris entre 1,0 et 20, de manière préférée entre 2,0 et 10. Par exemple, la section réactionnelle d'hydroconversion de l'étape b) du procédé comprend un catalyseur d'hydroconversion comprenant entre 0,5% et 10% en poids de nickel, de préférence entre 1% et 8% en poids de nickel, exprimé en oxyde de nickel NiO par rapport au poids total du catalyseur d'hydroconversion, et entre 1,0% et 30% en poids de molybdène, de préférence entre 3,0% et 29% en poids de molybdène, exprimé en oxyde de molybdène MoOs par rapport au poids total du catalyseur d'hydroconversion, sur un support minéral, de préférence sur un support d'alumine.

Le support dudit catalyseur d'hydroconversion est avantageusement choisi parmi l'alumine, la silice, les silices-alumines, la magnésie, les argiles et leurs mélanges. Ledit support peut en outre renfermer des composés dopants, notamment des oxydes choisis parmi l'oxyde de bore, en particulier le trioxyde de bore, la zircone, la cérine, l'oxyde de titane, l'anhydride phosphorique et un mélange de ces oxydes. De préférence, ledit catalyseur d'hydroconversion comprend un support d'alumine, de manière préférée un support d'alumine dopé avec du phosphore et éventuellement du bore. Lorsque l'anhydride phosphorique $P_2O_5$ est présent, sa concentration est inférieure à 10% en poids par rapport au poids de l'alumine et avantageusement d'au moins 0,001 % poids par rapport au poids total de l'alumine. Lorsque le trioxyde de bore $B_2O_5$ est présent, sa concentration est inférieure à 10% en poids par rapport au poids de l'alumine et avantageusement d'au moins 0,001 % par rapport au poids total de l'alumine. L'alumine utilisée peut être par exemple une alumine $\gamma$ (gamma) ou $\eta$ (éta).

Ledit catalyseur d'hydroconversion est par exemple sous forme d'extrudés ou de billes.

Avantageusement, ledit catalyseur d'hydroconversion utilisé dans l'étape b) du procédé présente une surface spécifique supérieure ou égale à 250 m$^2$/g, de préférence supérieure ou égale à 300 m$^2$/g. La surface spécifique dudit catalyseur d'hydroconversion est avantageusement inférieure ou égale à 800 m$^2$/g, de préférence inférieure ou égale à 600 m$^2$/g, en particulier inférieure ou égale à 400 m$^2$/g. La surface spécifique du catalyseur d'hydroconversion est mesurée par la méthode BET, c'est-à-dire la surface spécifique déterminée par adsorption d'azote conformément à la norme ASTM D 3663 établie à partir de la méthode BRUNAUER-EMMETT-TELLER décrite dans le périodique 'The Journal of the American Chemical Society", 6Q, 309 (1938). Une telle surface spécifique permet d'améliorer encore l'élimination des contaminants, en particulier des métaux comme le silicium.

Les catalyseurs d'hydroconversion se distinguent des catalyseurs d'hydrotraitement notamment par une porosité adaptée aux traitements des impuretés, notamment métalliques, et en particulier par la présence de macroporosité.

Selon un autre aspect de l'invention, le catalyseur d'hydroconversion tel que décrit plus haut comprend en outre un ou plusieurs composés organiques contenant de l'oxygène et/ou de l'azote et/ou du soufre. Un tel catalyseur est souvent désigné par le terme "catalyseur additivé". Généralement, le composé organique est choisi parmi un composé comportant une ou plusieurs fonctions chimiques choisies parmi une fonction carboxylique, alcool, thiol, thioéther, sulfone, sulfoxyde, éther, aldéhyde, cétone, ester, carbonate, amine, nitrile, imide, oxime, urée et amide ou encore les composés incluant un cycle furanique ou encore les sucres.

Etape b) d'hydroconversion en lit entraîné

Selon une deuxième variante, l'étape b) d'hydroconversion est mise en œuvre dans une section réactionnelle d'hydroconversion mettant en œuvre au moins un réacteur à lit entraîné, aussi appelé réacteur slurry selon la technologie anglo-saxonne. La charge, l'hydrogène et le catalyseur sont injectés par le bas et circulent en courant ascendant. L'effluent hydroconverti et l'hydrogène non consommé ainsi que le catalyseur sont soutirés par le haut. Les technologies d'hydroconversion en slurry utilisent un catalyseur dispersé sous forme de très petites particules, dont la taille est de quelques dizaines de microns ou moins (généralement 0,001 à 100 $\mu$m). Les catalyseurs, ou leurs précurseurs, sont injectés avec la charge à convertir à l'entrée des réacteurs. Les catalyseurs traversent les réacteurs avec les charges et les produits en cours de conversion, puis ils sont entraînés avec les produits de réaction hors des réacteurs. On les retrouve après séparation dans la fraction la plus lourde.

Le catalyseur en slurry est un catalyseur contenant de préférence au moins un élément choisi dans le groupe formé par Mo, Fe, Ni, W, Co, V, Ru. Ces catalyseurs sont généralement monométalliques ou bimétalliques (en combinant par exemple un élément du groupe VIIIB non-noble (Co,Ni,Fe) et un élément du groupe VIB (Mo,W).

Les catalyseurs utilisés peuvent être des poudres de solides hétérogènes (tels que des minerais naturels, du sulfate de fer, etc...), des catalyseurs dispersés issus de précurseurs solubles dans l'eau ("water soluble dispersed catalyst" selon la terminologie anglo-saxonne) tels que l'acide phosphomolybdique, le molybdate d'ammonium, ou un mélange d'oxyde Mo ou Ni avec de l'ammoniaque aqueux.

De préférence, les catalyseurs utilisés sont issus de précurseurs solubles dans une phase organique ("oil soluble dispersed catalyst" selon la terminologie anglo-saxonne). Les précurseurs sont des composés organométalliques tels que les naphténates de Mo, de Co, de Fe, ou de Ni ou tels que des composés multi-carbonyl de ces métaux, par exemple 2-ethylhexanoates de Mo ou Ni, acétylacétonates de Mo ou Ni, sels d'acides gras C7-C12 de Mo ou W, etc. Ils peuvent être utilisés en présence d'un agent tensio-actif pour améliorer la dispersion des métaux, lorsque le catalyseur est bimétallique.

Les catalyseurs se trouvent sous forme de particules dispersées, colloïdales ou non selon la nature du catalyseur. De tels précurseurs et catalyseurs utilisables dans le procédé selon l'invention sont largement décrits dans la littérature.

La concentration du catalyseur, exprimée en élément métallique est généralement entre 1 et 10000 ppm par rapport à la charge.

En général, les catalyseurs sont préparés avant d'être injectés dans la charge. Le procédé de préparation est adapté en fonction de l'état dans lequel se trouve le précurseur et de sa nature. Dans tous les cas, le précurseur est sulfuré (ex-situ ou in-situ) pour former le catalyseur dispersé dans la charge.

Pour le cas préféré des catalyseurs dits solubles dans l'huile, dans un procédé typique, le précurseur est mélangé à une charge carbonée (qui peut être une partie de la charge à traiter, une charge externe, une fraction recyclée...), le mélange est éventuellement séché au moins en partie, puis ou simultanément sulfuré par addition d'un composé soufré ($H_2S$ préféré) et chauffé. Les préparations de ces catalyseurs sont décrites dans l'art antérieur. Des additifs peuvent être ajoutés lors de la préparation du catalyseur ou au catalyseur en slurry avant qu'il soit injecté dans le réacteur. Ces additifs sont décrits dans la littérature.

Les additifs solides préférés sont des oxydes minéraux tels que l'alumine, la silice, des oxydes mixtes Al/Si, des catalyseurs usagés supportés (par exemple, sur alumine et/ou silice) contenant au moins un élément du groupe VIII (tel que Ni, Co) et/ou au moins un élément du groupe VIB (tel que Mo, VV). On citera par exemple les catalyseurs décrits dans la demande US2008/177124. Des solides carbonés à faible teneur d'hydrogène (par exemple 4% d'hydrogène) comme du coke, éventuellement prétraités, peuvent être également utilisés. On peut également utiliser des mélanges de tels additifs. Leurs tailles de particules sont de préférence inférieures à 1 mm. La teneur en éventuel additif solide présent à l'entrée de la zone réactionnelle d'hydroconversion en lit entraîné est comprise entre 0 et 10% pds préférentiellement entre 1 et 3% pds, et la teneur des solutions catalytiques est comprise entre 0 et 10% pds, de préférence entre 0 et 1% pds par rapport au poids de la charge injectée.

Lorsqu'on effectue l'étape b) d'hydroconversion dans un réacteur à lit entraîné, une étape de filtration afin de récupérer le catalyseur est nécessaire avant d'envoyer l'effluent hydroconverti dans l'étape c).

Etape b) d'hydroconversion en lit mobile

Selon une troisième variante, l'étape b) d'hydroconversion est mise en œuvre dans une section réactionnelle d'hydroconversion mettant en œuvre au moins un réacteur à lit mobile.

La charge et l'hydrogène peuvent circuler en écoulement ascendant dans les réacteurs à lit mobile (procédés à contre-courant) ou en écoulement descendant (procédés à co-courant). Le catalyseur s'écoule progressivement par gravité de haut en bas et en écoulement piston à l'intérieur de la zone catalytique. Il est soutiré par le bas par tout moyen approprié, par exemple un élévateur (dit « lift » selon la terminologie anglo-saxonne). Un dispositif en ligne assure le renouvellement semi continu du catalyseur des réacteurs à lit mobile : une partie du catalyseur usé est soutirée en fond de réacteur tandis que du catalyseur frais est introduit en tête de réacteur. La température y est contrôlée par des trempes inter ou intraréacteurs.

De préférence, on utilise des catalyseurs sphériques de diamètre compris entre 0,5 et 6 mm et de manière préférée entre 1 et 3 mm plutôt que des catalyseurs extrudés pour obtenir un meilleur écoulement. Lors du soutirage du catalyseur usagé en bas de réacteur, la totalité du lit catalytique se déplaçant en écoulement piston, se déplace vers le bas d'une hauteur correspondant au volume de catalyseur soutiré. Le taux d'expansion du lit catalytique fonctionnant en lit mobile est avantageusement inférieur à 15%, de préférence inférieur à 10%, de manière préférée inférieur à 5% et de manière plus préférée inférieur à 2%. Le taux d'expansion est mesuré selon une méthode connue de l'homme du métier.

Le catalyseur d'hydroconversion utilisé dans le lit mobile de l'étape b) du procédé selon l'invention est avantageusement un catalyseur comprenant un support, de préférence amorphe et de manière très préférée de l'alumine et au moins un métal du groupe VIII choisi parmi le nickel et le cobalt et de préférence le nickel, ledit élément du groupe VIII étant de préférence utilisé en association avec au moins un métal du groupe VIB choisi parmi le molybdène et le tungstène et de préférence, le métal du groupe VIB est le molybdène. De préférence, le catalyseur d'hydroconversion comprend le nickel en tant qu'élément du groupe VIII et le molybdène en tant qu'élément du groupe VIB. La teneur nickel est avantageusement comprise entre 0,5 à 10 % exprimée en poids d'oxyde de nickel (NiO) et de préférence entre 1 à 6 % poids, et la teneur en molybdène est avantageusement comprise entre 1 et 30 % exprimée en poids de trioxyde de molybdène (MoOs), et de préférence entre 4 et 20 % poids, les pourcentages étant exprimés en pourcentage poids par rapport au poids total du catalyseur. Ce catalyseur est avantageusement sous forme d'extrudés ou de billes. Ce catalyseur peut également avantageusement contenir du phosphore et de préférence une teneur en anhydride

phosphorique $P_2O_5$ inférieure à 20% et de manière préférée inférieure à 10% poids, les pourcentages étant exprimés en pourcentage poids par rapport au poids total du catalyseur. Le catalyseur peut aussi être un catalyseur additivé d'un composé organique tel que décrit ci-dessus.

Selon une autre variante encore, l'étape b) d'hydroconversion peut être mise en œuvre dans une section réactionnelle d'hydroconversion mettant en œuvre une combinaison d'au moins un réacteur à lit bouillonnant, d'au moins un réacteur à lit en lit entraîné et/ou d'au moins un réacteur à lit mobile et ceci dans n'importe quel ordre.

De préférence, l'étape b) est mise en œuvre dans une section réactionnelle d'hydroconversion mettant en œuvre au moins un réacteur à lit bouillonnant.

**Etape c) de séparation**

Selon l'invention, le procédé de traitement comprend une étape c) de séparation, avantageusement mise en œuvre dans au moins une section de lavage/séparation, alimentée au moins par l'effluent hydroconverti issu de l'étape b) et une solution aqueuse, pour obtenir au moins un effluent gazeux, un effluent aqueux et un effluent hydrocarboné.

L'effluent gazeux obtenu à l'issu de l'étape c) comprend avantageusement de l'hydrogène, de préférence comprend au moins 90% volume, de préférence au moins 95% volume, d'hydrogène. Avantageusement, ledit effluent gazeux peut au moins en partie être recyclé vers les étapes a) d'hydrogénation sélective et/ou b) d'hydroconversion et/ou e) d'hydrotraitement et/ou e') d'hydrocraquage, le système de recyclage pouvant comprendre une section de purification.

L'effluent aqueux obtenu à l'issu de l'étape c) comprend avantageusement des sels d'ammonium et/ou de l'acide chlorhydrique. L'effluent aqueux peut être recyclé dans l'étape c).

L'étape c) de séparation permet en particulier d'éliminer les sels de chlorure d'ammonium, qui se forment par réaction entre les ions chlorure, libérés par l'hydrogénation des composés chlorés sous forme HCl notamment lors de l'étape b) puis dissolution dans l'eau, et les ions ammonium, générés par l'hydrogénation des composés azotés sous forme de $NH_3$ notamment lors de l'étape b) et/ou apportés par injection d'une amine puis dissolution dans l'eau, et ainsi de limiter les risques de bouchage, en particulier dans les lignes de transfert et/ou dans les sections du procédé de l'invention et/ou les lignes de transfert vers le vapocraqueur, dû à la précipitation des sels de chlorure d'ammonium. Il permet aussi d'éliminer l'acide chlorhydrique formé par la réaction des ions hydrogène et des ions chlorures.

En fonction de la teneur en composés chlorés dans la charge initiale à traiter, un flux contenant une amine telle que par exemple la monoéthanolamine, la diéthanolamine et/ou la monodiéthanolamine peut être injecté en amont de l'étape a) d'hydrogénation sélective, entre l'étape a) d'hydrogénation sélective et l'étape b) d'hydroconversion et/ou entre l'étape b) d'hydroconversion et l'étape c) de séparation, de préférence en amont de l'étape a) d'hydrogénation sélective lorsqu'elle est présente, afin d'assurer une quantité suffisante en ions ammonium pour combiner les ions chlorure formés lors de l'étape d'hydroconversion, permettant ainsi de limiter la formation d'acide chlorhydrique et ainsi de limiter la corrosion en aval de la section de séparation.

Avantageusement, l'étape c) de séparation comprend une injection d'une solution aqueuse, de préférence une injection d'eau, dans l'effluent hydroconverti issu de l'étape b), en amont de la section de lavage/séparation, de manière à dissoudre au moins en partie des sels de chlorure d'ammonium et/ou de l'acide chlorhydrique et améliorer ainsi l'élimination des impuretés chlorées et réduire les risques de bouchages dus à une accumulation des sels de chlorure d'ammonium.

L'étape c) de séparation est avantageusement opérée à une température comprise entre 50 et 450°C, préférentiellement entre 100 et 440°C, de manière préférée entre 200 et 420°C. Il est important d'opérer dans cette gamme de température (et donc de ne pas trop refroidir l'effluent hydroconverti) au risque de bouchage dans les lignes dû à la précipitation des sels de chlorure d'ammonium. Avantageusement, l'étape c) de séparation est opérée à une pression proche de celle mise en œuvre dans les étapes a) et/ou b), de préférence entre 1,0 et 20,0 MPa, de manière à faciliter le recyclage d'hydrogène.

La section de lavage/séparation de l'étape c) peut au moins en partie être réalisée dans des équipements de lavage et de séparation communs ou distincts, ces équipements étant bien connus (ballons séparateurs pouvant opérés à différentes pressions et températures, pompes, échangeurs de chaleurs, colonnes de lavage, etc.).

Dans un mode de réalisation de l'invention, l'étape c) de séparation comprend l'injection d'une solution aqueuse dans l'effluent hydroconverti issu de l'étape b), suivi de la section de lavage/séparation comprenant avantageusement une phase de séparation permettant d'obtenir au moins un effluent aqueux chargé en sels d'ammonium, un effluent hydrocarboné liquide lavé et un effluent gazeux partiellement lavé. L'effluent aqueux chargé en sels d'ammonium et l'effluent hydrocarboné liquide lavé peuvent ensuite être séparés dans un ballon décanteur afin d'obtenir ledit effluent hydrocarboné et ledit effluent aqueux. Ledit effluent gazeux partiellement lavé peut parallèlement être introduit dans une colonne de lavage où il circule à contrecourant d'un flux aqueux, de préférence de même nature que la solution aqueuse injectée dans l'effluent hydroconverti, ce qui permet d'éliminer au moins en partie, de préférence en totalité, l'acide chlorhydrique contenu dans l'effluent gazeux partiellement lavé et d'obtenir ainsi ledit effluent gazeux, comprenant de préférence essentiellement de l'hydrogène, et un flux aqueux acide. Ledit effluent aqueux issu du ballon décanteur peut éventuellement être mélangé avec ledit flux aqueux acide, et être utilisé, éventuellement en mélange avec ledit flux aqueux acide dans un circuit de recyclage d'eau pour alimenter l'étape c) de séparation en ladite solution aqueuse en amont de la section de lavage/séparation et/ou en ledit flux aqueux dans la colonne de lavage. Ledit circuit de recyclage d'eau peut comporter un appoint d'eau et/ou d'une solution basique et/ou une purge permettant d'évacuer les sels dissous.

Dans un autre mode de réalisation de l'invention, l'étape c) de séparation peut comprendre avantageusement une section de lavage/séparation à « haute pression » qui opère à une pression proche de la pression de l'étape a) d'hydrogénation sélective et/ou de l'étape b) d'hydroconversion, de préférence entre 1,0 et 20,0 MPa, afin de faciliter le recyclage d'hydrogène. Cette section « haute pression » de l'étape c) peut être complétée par une section « basse pression », de préférence une pression généralement comprise entre 0,5 et 10,0 MPa, afin d'obtenir une fraction liquide hydrocarbonée dépourvue d'une partie des gaz dissous à haute pression et destinée à être traitée envoyée dans l'étape d) de fractionnement.

La ou les fractions gaz issue(s) de l'étape c) de séparation peut (peuvent) faire l'objet de purification(s) et de séparation(s) complémentaire(s) en vue de récupérer au moins un gaz riche en hydrogène pouvant être recyclé en amont des étapes a) et/ou b) et/ou e) et/ou e'), et des hydrocarbures légers, notamment de l'éthane, du propane et du butane, qui peuvent avantageusement être envoyés séparément ou en mélange dans un ou des fours de l'étape h) de vapocraquage.

L'effluent hydrocarboné issu de l'étape c) de séparation est envoyé, en partie ou en totalité, de manière préférée en totalité, vers l'étape d) de fractionnement.

**Etape d) de fractionnement**

Le procédé selon l'invention comprend une étape de fractionnement de tout ou partie, de manière préférée de la totalité, de l'effluent hydrocarboné issu de l'étape c), pour obtenir au moins un flux gazeux, une coupe hydrocarbonée comprenant des composés ayant un point d'ébullition inférieur ou égal à 385°C et une coupe hydrocarbonée comprenant des composés ayant un point d'ébullition supérieur à 385°C.

L'étape d) permet en particulier d'éliminer les gaz dissous dans l'effluent liquide hydrocarboné, comme par exemple de l'ammoniac, de l'hydrogène sulfuré et des hydrocarbures légers ayant 1 à 4 atomes de carbone.

L'étape d) de fractionnement est avantageusement opérée à une pression inférieure ou égale à 1,0 MPa abs., de préférence entre 0,1 et 1,0 MPa abs.

Selon un mode de réalisation, l'étape d) peut être opérée dans une section comprenant avantageusement au moins une colonne de stripage équipée d'un circuit de reflux comprenant un ballon de reflux. Ladite colonne de stripage est alimentée par l'effluent liquide hydrocarboné issu de l'étape c) et par un flux de vapeur d'eau. L'effluent liquide hydrocarboné issu de l'étape c) peut être éventuellement réchauffé avant l'entrée dans la colonne de stripage. Ainsi, les composés les plus légers sont entrainés en tête de colonne et dans le circuit de reflux comprenant un ballon de reflux dans lequel s'opère une séparation gaz/liquide. La phase gazeuse qui comprend les hydrocarbures légers, est soutirée du ballon de reflux, en un flux gazeux. La coupe comprenant des composés ayant un point d'ébullition inférieur ou égal à 385°C est avantageusement soutirée du ballon de reflux. La coupe hydrocarbonée comprenant des composés ayant un point d'ébullition supérieur à 385°C est avantageusement soutirée en fond de colonne de stripage.

Selon d'autres modes de réalisation, l'étape d) de fractionnement peut mettre en œuvre une colonne de stripage suivie d'une colonne de distillation ou uniquement une colonne de distillation.

La coupe comprenant des composés ayant un point d'ébullition inférieur ou égal à 385°C (coupe naphta et coupe diesel) est envoyée, en tout ou partie, vers l'étape e) d'hydrotraitement.

La coupe comprenant des composés ayant un point d'ébullition supérieur à 385°C (huiles non converties) est avantageusement au moins en partie recyclée dans l'étape b) d'hydroconversion. Elle peut également être brulée pour produire de la chaleur et/ou de l'électricité.

Une purge peut être installée sur le recycle de ladite coupe comprenant des composés ayant un point d'ébullition supérieur à 385°C issu de l'étape d). En fonction des conditions opératoires du procédé, ladite purge peut être comprise entre 0 et 50% poids de ladite coupe issu de l'étape d), et de préférence entre 5% et 20%poids.

La ou les fractions gaz issue(s) de l'étape d) de fractionnement peut (peuvent) faire l'objet de purification(s) et de séparation(s) complémentaire(s) en vue de récupérer au moins des hydrocarbures légers, notamment de l'éthane, du propane et du butane, qui peuvent avantageusement être envoyés séparément ou en mélange dans un ou des fours de l'étape h) de vapocraquage.

**Etape e) d'hydrotraitement**

Selon l'invention, le procédé de traitement comprend une étape e) d'hydrotraitement mise en œuvre dans une section réactionnelle d'hydrotraitement, mettant en œuvre au moins un réacteur à lit fixe ayant n lits catalytiques, n étant un nombre entier supérieur ou égal à 1, comprenant chacun au moins un catalyseur d'hydrotraitement, ladite section réactionnelle d'hydrotraitement étant alimentée par au moins une partie de ladite coupe hydrocarbonée comprenant des composés ayant un point d'ébullition inférieur ou égal à 385°C issue de l'étape d) et un flux gazeux comprenant de l'hydrogène, pour obtenir un effluent hydrotraité.

Avantageusement, l'étape e) met en œuvre les réactions d'hydrotraitement bien connues de l'homme du métier, et plus particulièrement des réactions d'hydrotraitement telles que l'hydrogénation des aromatiques, l'hydrodésulfuration et l'hydrodéazotation. De plus, l'hydrogénation des oléfines et des composés halogénés restants ainsi que l'hydrodémétallation sont poursuivies.

Avantageusement, ladite étape e) est mise en œuvre dans une section réactionnelle d'hydrotraitement comprenant au moins un, de préférence entre un et cinq, réacteur(s) à lit fixe ayant n lits catalytiques, n étant un nombre entier supérieur ou égal à un, de préférence compris entre un et dix, de manière préférée compris entre deux et cinq, le(s)dit(s) lit(s) comprenant chacun au moins un, et de préférence pas plus de dix, catalyseur(s) d'hydrotraitement. Lorsqu'un réacteur comprend plusieurs lits catalytiques, c'est-à-dire au moins deux, de préférence entre deux et dix, de manière préférée entre deux et cinq lits catalytiques, lesdits lits catalytiques sont disposés en série dans ledit réacteur.

Ladite section réactionnelle d'hydrotraitement est alimentée par au moins une partie de ladite coupe hydrocarbonée comprenant des composés ayant un point d'ébullition inférieur ou égal à 385°C issue de l'étape d) et un flux gazeux comprenant de l'hydrogène, avantageusement au niveau du premier lit catalytique du premier réacteur en fonctionnement.

Ladite section réactionnelle d'hydrotraitement de l'étape e) peut également être alimentée par au moins une fraction du flux de recycle, avantageusement issu de l'étape g) optionnelle. La(les)dite(s) fraction(s) dudit flux de recycle ou la totalité du flux de recycle peu(ven)t être introduite(s) dans ladite section réactionnelle d'hydrotraitement en mélange avec ladite coupe hydrocarbonée comprenant des composés ayant un point d'ébullition inférieur ou égal à 385°C issue de l'étape d) ou séparément. La(les)dite(s) fraction(s) dudit flux de recycle ou la totalité du flux de recycle peu(ven)t être introduite(s) dans ladite section réactionnelle d'hydrotraitement au niveau d'un ou de plusieurs lits catalytiques de ladite section réactionnelle d'hydrotraitement de l'étape e). L'introduction d'au moins une fraction dudit flux de recycle permet avantageusement de diluer les impuretés encore présentes dans l'effluent hydrogéné et de contrôler la température, en particulier de limiter l'augmentation de température, dans le ou les lit(s) catalytique(s) de la section réactionnelle d'hydrotraitement qui met en œuvre des réactions fortement exothermiques.

Ladite section réactionnelle d'hydrotraitement est avantageusement mise en œuvre à une température d'hydrotraitement entre 250 et 430°C, de préférence entre 300 et 400°C, à une pression partielle d'hydrogène entre 1,0 et 20,0 MPa abs., de préférence entre 3,0 et 15,0 MPa abs, et à une vitesse volumique horaire (VVH) entre 0,1 et 10,0 $h^{-1}$, de préférence entre 0,1 et 5,0 $h^{-1}$, préférentiellement entre 0,2 et 2,0 $h^{-1}$, de manière préférée entre 0,2 et 1,0 $h^{-1}$. Selon l'invention, la « température d'hydrotraitement » correspond à une température moyenne dans la section réactionnelle d'hydrotraitement de l'étape e). En particulier, elle correspond à la Weight Average Bed Température (WABT) selon le terme anglo-saxon consacré, bien connue de l'Homme du métier. La température d'hydrotraitement est avantageusement déterminée en fonction des systèmes catalytiques, des équipements, de la configuration de ceux-ci, utilisés. Par exemple, la température d'hydrotraitement (ou WABT) est calculée de la manière suivante :

$$WABT = (T_{entrée} + 2x\ T_{sortie})/3$$

avec $T_{entrée}$ : la température de l'effluent ayant un point d'ébullition inférieur ou égal à 385°C en entrée de la section réactionnelle d'hydrotraitement, $T_{sortie}$ : la température de l'effluent en sortie de section réactionnelle d'hydrotraitement.

La vitesse volumique horaire (VVH) est définie ici comme le ratio entre le débit volumique horaire de la coupe hydrocarbonée comprenant des composés ayant un point d'ébullition inférieur ou égal à 385°C issue de l'étape d) par volume de catalyseur(s). La couverture en hydrogène dans l'étape e) est avantageusement comprise entre 50 et 2000 $Nm^3$ d'hydrogène par $m^3$ de charge qui alimente l'étape e), et de préférence entre 100 et 1000 $Nm^3$ d'hydrogène par $m^3$ de charge qui alimente l'étape e), de manière préférée entre 120 et 800 $Nm^3$ d'hydrogène par $m^3$ de charge qui alimente l'étape e). La couverture en hydrogène est définie ici comme le rapport du débit volumique d'hydrogène pris dans les conditions normales de température et pression par rapport au débit volumique de charge qui alimente l'étape e) (en normaux $m^3$, noté $Nm^3$, de $H_2$ par $m^3$ de charge fraîche). L'hydrogène peut être constitué d'un appoint et/ou d'hydrogène recyclé issu en particulier de l'étape c) de séparation.

De préférence, un flux gazeux supplémentaire comprenant de l'hydrogène est avantageusement introduit en entrée de chaque réacteur, en particulier fonctionnant en série, et/ou en entrée de chaque lit catalytique à partir du second lit catalytique de la section réactionnelle d'hydrotraitement. Ces flux gazeux supplémentaires sont appelés encore flux de refroidissement. Ils permettent de contrôler la température dans le réacteur d'hydrotraitement dans lequel les réactions mises en œuvre sont généralement très exothermiques.

Avantageusement, ledit catalyseur d'hydrotraitement utilisé dans ladite étape e) peut être choisi parmi des catalyseurs connus d'hydrodémétallation, d'hydrotraitement, de captation du silicium, utilisés notamment pour le traitement des coupes pétrolières, et leurs combinaisons. Des catalyseurs d'hydrodémétallation connus sont par exemple ceux décrits dans les brevets EP 0113297, EP 0113284, US 5221656, US 5827421, US 7119045, US 5622616 et US 5089463. Des catalyseurs d'hydrotraitement connus sont par exemple ceux décrits dans les brevets EP 0113297, EP 0113284, US 6589908, US 4818743 ou US 6332976. Des catalyseurs de captation du silicium connus sont par exemple ceux décrits dans les demandes de brevets CN 102051202 et US 2007/080099.

En particulier, ledit catalyseur d'hydrotraitement comprend un support, de préférence minéral, et au moins un élément métallique ayant une fonction hydro-déshydrogénante. Ledit élément métallique ayant une fonction hydro-déshydrogénante comprend avantageusement au moins un élément du groupe VIII, de préférence choisi dans le groupe constitué par le nickel et le cobalt, et/ou au moins un élément du groupe VIB, de préférence choisi dans le groupe constitué par le molybdène et le tungstène. La teneur totale en oxydes des éléments métalliques des groupes VIB et VIII est de préférence entre 0,1% et 40% en poids, préférentiellement de 5% à 35% en poids, par rapport au poids total du catalyseur. Le rapport pondéral exprimé en oxyde métallique entre le métal (ou les métaux) du groupe VIB par rapport au métal (ou aux métaux) du groupe VIII est de préférence compris entre 1,0 et 20, de manière préférée entre 2,0 et 10. Par exemple, la section réactionnelle d'hydrotraitement de l'étape b) du procédé comprend un catalyseur d'hydrotraitement comprenant entre 0,5% et 10% en poids de nickel, de préférence entre 1% et 8% en poids de nickel, exprimé en oxyde de nickel NiO par rapport au poids total du catalyseur d'hydrotraitement, et entre 1,0% et 30% en poids total de molybdène et/ou de tungstène, de préférence entre 3,0% et 29% en poids, exprimé en oxyde de molybdène $MoO_3$ ou en oxyde de tungstène $WO_3$ par rapport au poids total du catalyseur d'hydrotraitement, sur un support minéral.

Le support dudit catalyseur d'hydrotraitement est avantageusement choisi parmi l'alumine, la silice, les silices-alumines, la magnésie, les argiles et leurs mélanges. Ledit support peut en outre renfermer avantageusement des composés dopants, notamment des oxydes choisis parmi l'oxyde de bore, en particulier le trioxyde de bore, la zircone, la cérine, l'oxyde de titane, l'anhydride phosphorique et un mélange de ces oxydes. De préférence, ledit catalyseur d'hydrotraitement comprend un support d'alumine, de manière préférée un support d'alumine dopé avec du phosphore et éventuellement du bore. Lorsque l'anhydride phosphorique $P_2O_5$ est présent, sa concentration est inférieure à 10% en poids par rapport au poids de l'alumine et avantageusement d'au moins 0,001 % poids par rapport au poids total de l'alumine. Lorsque le trioxyde de bore $B_2O_5$ est présent, sa concentration est inférieure à 10% en poids par rapport au poids de l'alumine et avantageusement d'au moins 0,001 % par rapport au poids total de l'alumine. L'alumine utilisée peut être par exemple une alumine $\gamma$ (gamma) ou $\eta$ (éta).

Ledit catalyseur d'hydrotraitement est par exemple sous forme d'extrudés.

Avantageusement, ledit catalyseur d'hydrotraitement utilisé dans l'étape e) du procédé présente une surface spécifique supérieure ou égale à 250 m$^2$/g, de préférence supérieure ou égale à 300 m$^2$/g. La surface spécifique dudit catalyseur d'hydrotraitement est avantageusement inférieure ou égale à 800 m$^2$/g, de préférence inférieure ou égale à 600 m$^2$/g, en particulier inférieure ou égale à 400 m$^2$/g. La surface spécifique du catalyseur d'hydrotraitement est mesurée par la méthode BET, c'est-à-dire la surface spécifique déterminée par adsorption d'azote conformément à la norme ASTM D 3663 établie à partir de la méthode BRUNAUER-EMMETT-TELLER décrite dans le périodique 'The Journal of the American Chemical Society", 6Q, 309 (1938). Une telle surface spécifique permet d'améliorer encore l'élimination des contaminants, en particulier des métaux comme le silicium.

Selon un autre aspect de l'invention, le catalyseur d'hydrotraitement tel que décrit plus haut comprend en outre un ou plusieurs composés organiques contenant de l'oxygène et/ou de l'azote et/ou du soufre. Un tel catalyseur est souvent désigné par le terme "catalyseur additivé". Généralement, le composé organique est choisi parmi un composé comportant une ou plusieurs fonctions chimiques choisies parmi une fonction carboxylique, alcool, thiol, thioéther, sulfone, sulfoxyde, éther, aldéhyde, cétone, ester, carbonate, amine, nitrile, imide, oxime, urée et amide ou encore les composés incluant un cycle furanique ou encore les sucres.

La préparation des catalyseurs des étapes a), b) ou e) ou encore e') (décrite ci-dessous) est connue et comprend lorsqu'il s'agit de catalyseurs supportés, généralement une étape d'imprégnation des métaux du groupe VIII et du groupe VIB lorsqu'il est présent, et éventuellement du phosphore et/ou du bore sur le support, suivie d'un séchage, puis éventuellement d'une calcination. Dans le cas de catalyseur additivé, la préparation se fait généralement par simple séchage sans calcination après introduction du composé organique. On entend ici par calcination un traitement thermique sous un gaz contenant de l'air ou de l'oxygène à une température supérieure ou égale à 200°C. Avant leur utilisation dans une étape du procédé, les catalyseurs sont généralement soumis à une sulfuration afin de former l'espèce active.

Dans un mode de réalisation préféré de l'invention, ladite section réactionnelle d'hydrotraitement comprend plusieurs réacteurs à lit fixe, préférentiellement entre deux et cinq, très préférentiellement entre deux et quatre, réacteurs à lit fixe, ayant chacun n lits catalytiques, n étant un nombre entier supérieur ou égal à un, de préférence compris entre un et dix, de manière préférée compris entre deux et cinq, et fonctionnant avantageusement en série et/ou en parallèle et/ou en mode permutable (ou PRS) et/ou en mode « swing ». Les différents modes de fonctionnement éventuels, mode PRS (ou lead and lag) et mode swing, et sont bien connus de l'Homme du métier et sont avantageusement définis plus haut. L'avantage d'une section réactionnelle d'hydrotraitement comprenant plusieurs réacteurs réside dans un traitement optimisé de la coupe hydrocarbonée comprenant des composés ayant un point d'ébullition inférieur ou égal à 385°C issue de l'étape d), tout en permettant de diminuer les risques de colmatage du ou des lits catalytiques et donc d'éviter l'arrêt de l'unité dû au colmatage.

Selon un mode de réalisation très préféré de l'invention, ladite section réactionnelle d'hydrotraitement comprend au moins un réacteur à lit fixe, de préférence consiste en un réacteur ou deux réacteurs en série, le ou lesdits réacteurs à lit fixe ayant entre 1 et 5 lits catalytiques disposés en série et comprenant chacun entre un et dix catalyseur(s) d'hydrotraitement dont au moins un desdits catalyseurs d'hydrotraitement comprend avantageusement un support et au moins un élément métallique comprenant de préférence au moins un élément du groupe VIII, de préférence choisi parmi le nickel et le cobalt, et/ou au moins un élément du groupe VIB, de préférence choisi parmi le molybdène et le tungstène.

Eventuellement, l'étape e) peut mettre en œuvre une section de chauffe située en amont de la section réactionnelle d'hydrotraitement et dans laquelle la coupe hydrocarbonée comprenant des composés ayant un point d'ébullition inférieur ou égal à 385°C issue de l'étape d) est chauffée pour atteindre une température adaptée pour l'hydrotraitement, c'est-à-dire une température comprise entre 250 et 430°C. Ladite éventuelle section de chauffe peut ainsi comprendre un ou plusieurs échangeurs, permettant de préférence un échange de chaleur entre la coupe hydrocarbonée comprenant des composés ayant un point d'ébullition inférieur ou égal à 385°C issue de l'étape d) et l'effluent d'hydrotraité, et/ou un four de préchauffe.

L'étape e) d'hydrotraitement permet avantageusement un traitement optimisé de la coupe hydrocarbonée comprenant des composés ayant un point d'ébullition inférieur ou égal à 385°C issue de l'étape d). Elle permet notamment de se débarrasser des impuretés restantes, notamment d'éliminer les composés soufrés et les composés azotés ainsi que les des métaux restants.

**Etape** e') **d'hydrocraquage (optionnelle)**

Selon une variante, le procédé de l'invention peut comprendre une étape e') d'hydrocraquage effectuée soit directement après l'étape e) d'hydrotraitement, soit après l'étape f) de séparation sur une coupe lourde (coupe diesel).

La coupe comprenant des composés ayant un point d'ébullition inférieur ou égal à 385°C comprend une coupe comprenant des composés ayant un point d'ébullition inférieur à 175°C (coupe naphta) et une coupe comprenant des composés ayant un point d'ébullition supérieur à 175°C et inférieur à 385°C (coupe diesel). Lorsqu'on souhaite minimiser le rendement de la coupe diesel et maximiser le rendement de la coupe naphta, on peut transformer la coupe diesel au moins en partie en coupe naphta par hydrocraquage, voire une partie de la coupe naphta lourde en naphta léger, coupe généralement favorisée pour une unité de vapocraquage.

Ainsi, le procédé de l'invention peut comprendre une étape e') d'hydrocraquage mise en œuvre dans une section réactionnelle d'hydrocraquage, mettant en œuvre au moins un lit fixe ayant n lits catalytiques, n étant un nombre entier supérieur ou égal à 1, comprenant chacun au moins un catalyseur d'hydrocraquage, ladite section réactionnelle d'hydrocraquage étant alimentée par ledit effluent hydrotraité issu de l'étape e) et/ou par la coupe diesel comprenant des composés ayant un point d'ébullition supérieur à 175°C et inférieur à 385°C issue de l'étape f) et un flux gazeux comprenant de l'hydrogène, ladite section réactionnelle d'hydrocraquage étant mise en œuvre à une température entre 250 et 450°C, une pression partielle d'hydrogène entre 1,5 et 20,0 MPa abs. et une vitesse volumique horaire entre 0,1 et 10,0 h$^{-1}$, pour obtenir un effluent hydrocraqué qui est envoyé dans l'étape f) de séparation.

Avantageusement, l'étape e') met en œuvre les réactions d'hydrocraquage bien connues de l'homme du métier, et permet plus particulièrement de convertir les composés lourds, par exemple des composés ayant un point d'ébullition supérieur à 175°C en composés ayant un point d'ébullition inférieur ou égal à 175°C contenus dans l'effluent hydrotraité issu de l'étape b). D'autres réactions, comme l'hydrogénation des oléfines, des aromatiques, l'hydrodémétallation, l'hydrodésulfuration, l'hydrodéazotation, etc. peuvent se poursuivent.

Avantageusement, ladite étape e') est mise en œuvre dans une section réactionnelle d'hydrocraquage comprenant au moins un, de préférence entre un et cinq lit fixe ayant n lits catalytiques, n étant un nombre entier supérieur ou égal à un, de préférence compris entre un et dix, de manière préférée compris entre deux et cinq, le(s)dit(s) lit(s) comprenant chacun au moins un, et de préférence pas plus de dix, catalyseur(s) d'hydrocraquage.

L'étape e) d'hydrotraitement et l'étape e') d'hydrocraquage peuvent avantageusement être réalisées dans un même réacteur ou dans des réacteurs différents. Dans le cas où elles sont réalisées dans un même réacteur, le réacteur comprend plusieurs lits catalytiques, les premiers lits catalytiques comprenant le ou les catalyseurs d'hydrotraitement et les lits catalytiques suivants comprenant le ou les catalyseurs d'hydrocraquage.

Ladite section réactionnelle d'hydrocraquage est avantageusement mise en œuvre à une température d'hydrotraitement entre 250 et 450°C, de préférence entre 320 et 430°C, à une pression partielle d'hydrogène entre 1,5 et 20 MPa abs, et à une vitesse volumique horaire (VVH) entre 0,1 et 10,0 h$^{-1}$, de préférence entre 0,1 et 5,0 h$^{-1}$, préférentiellement entre 0,2 et 4 h$^{-1}$. Selon l'invention, la « température d'hydrocraquage » correspond à une température moyenne dans la section réactionnelle d'hydrocraquage de l'étape c). En particulier, elle correspond à la Weight Average Bed Température (WABT) selon le terme anglo-saxon consacré, bien connue de l'Homme du métier. La température d'hydrocraquage est avantageusement déterminée en fonction des systèmes catalytiques, des équipements, de la configuration de ceux-ci, utilisés. Par exemple, la température d'hydrocraquage (ou WABT) est calculée de la manière suivante :

$$WABT = (T_{entrée} + 2x\ T_{sortie})/3$$

avec $T_{entrée}$ : la température de l'effluent hydrogéné en entrée de la section réactionnelle d'hydrocraquage, $T_{sortie}$ : la température de l'effluent en sortie de section réactionnelle d'hydrocraquage.

La vitesse volumique horaire (VVH) est définie ici comme le ratio entre le débit volumique horaire de l'effluent hydrotraité issu de l'étape e) par volume de catalyseur(s). La couverture en hydrogène dans l'étape e') est avantageusement comprise entre 80 et 2000 Nm$^3$ d'hydrogène par m$^3$ de charge qui alimente l'étape e'), et de préférence entre 200 et 1800 Nm$^3$ d'hydrogène par m$^3$ de charge qui alimente l'étape e'). La couverture en hydrogène est définie ici comme le rapport du débit volumique d'hydrogène pris dans les conditions normales de température et pression par rapport au débit volumique de charge qui alimente l'étape e') (en normaux m$^3$, noté Nm$^3$, de H$_2$ par m$^3$ de charge). L'hydrogène peut être constitué d'un appoint et/ou d'hydrogène recyclé issu en particulier des étapes c) et d) de séparation.

De préférence, un flux gazeux supplémentaire comprenant de l'hydrogène est avantageusement introduit en entrée de chaque réacteur, en particulier fonctionnant en série, et/ou en entrée de chaque lit catalytique à partir du second lit catalytique de la section réactionnelle d'hydrocraquage. Ces flux gazeux supplémentaires sont appelés encore flux de refroidissement. Ils permettent de contrôler la température dans le réacteur d'hydrocraquage dans lequel les réactions mises en œuvre sont généralement très exothermiques.

L'étape e') d'hydrocraquage peut être effectuée en une ou deux étapes. Lorsqu'elle est effectuée en deux étapes, on effectue une séparation de l'effluent issue de la première étape d'hydrocraquage e') permettant d'obtenir une coupe comprenant des composés ayant un point d'ébullition supérieur à 175°C (coupe diesel), laquelle est introduite dans la deuxième étape d'hydrocraquage. Cette configuration est particulièrement adaptée lorsqu'on souhaite produire uniquement une coupe naphta. Les conditions opératoires et catalyseurs utilisés dans les deux étapes d'hydrocraquage peuvent être identiques ou différentes.

Ces conditions opératoires utilisées dans l'étape e') du procédé selon l'invention permettent généralement d'atteindre des conversions par passe, en produits ayant au moins 80% en volume de composés ayant des points d'ébullition inférieurs ou égale à 175°C, de préférence inférieurs à 160°C et de manière préférée inférieurs à 150°C, supérieures à 15% poids et de manière encore plus préférée comprises entre 20 et 80% poids. Lorsque le procédé est effectué en deux étapes d'hydrocraquage, la conversion par passe dans la deuxième étape est maintenue modérée afin de maximiser la sélectivité en composés de la coupe naphta (ayant un point d'ébullition inférieur ou égal à 175°C, en particulier entre 80 et inférieur ou égal à 175°C). La conversion par passe est limitée par l'utilisation d'un taux de recycle élevé sur la boucle de la deuxième étape d'hydrocraquage. Ce taux est défini comme le ratio entre le débit d'alimentation de l'étape f) et le débit de la charge de l'étape a), préférentiellement ce ratio est compris entre 0,2 et 4, de manière préférée entre 0,5 et 2,5.

L'étape e') d'hydrocraquage ne permet ainsi forcément de transformer tous les composés ayant un point d'ébullition supérieur à 175°C (coupe diesel) en composés ayant un point d'ébullition inférieur ou égal à 175°C (coupe naphta). Après l'étape de fractionnement f), il peut rester donc une proportion plus ou moins importante de composés ayant un point d'ébullition supérieur à 175°C. Pour augmenter la conversion, au moins une partie de cette coupe non convertie peut être recyclée comme décrit ci-dessous à l'étape e'). Une autre partie peut être purgée. En fonction des conditions opératoires du procédé, ladite purge peut être comprise entre 0 et 10% poids de la coupe comprenant des composés ayant un point d'ébullition supérieur à 175°C par rapport à la charge entrante, et de préférence entre 0,5% et 5% poids.

Conformément à l'invention, l'étape e') d'hydrocraquage opère en présence d'au moins un catalyseur d'hydrocraquage.

Le ou les catalyseur(s) d'hydrocraquage utilisé(s) dans l'étape e') d'hydrocraquage sont des catalyseurs classiques d'hydrocraquage connus de l'Homme du métier, de type bifonctionnel associant une fonction acide à une fonction hydro-déshydrogénante et éventuellement au moins une matrice liante. La fonction acide est apportée par des supports de grande surface (150 à 800 m$^2$/g généralement) présentant une acidité superficielle, telles que les alumines halogénées (chlorées ou fluorées notamment), les combinaisons d'oxydes de bore et d'aluminium, les silice-alumines amorphes et les zéolithes. La fonction hydro-déshydrogénante est apportée par au moins un métal du groupe VIB de la classification périodique et/ou au moins un métal du groupe VIII.

De préférence, le ou les catalyseurs d'hydrocraquage utilisés dans l'étape e') comprennent une fonction hydro-déshydrogénante comprenant au moins un métal du groupe VIII choisi parmi le fer, le cobalt, le nickel, le ruthénium, le rhodium, le palladium et le platine, et de préférence parmi le cobalt et le nickel. De préférence, le(s)dit(s) catalyseurs comprennent également au moins un métal du groupe VIB choisi parmi le chrome, le molybdène et le tungstène, seul ou en mélange, et de préférence parmi le molybdène et le tungstène. Des fonctions hydro-déshydrogénantes de type NiMo, NiMoW, NiW sont préférées.

De préférence, la teneur en métal du groupe VIII dans le ou les catalyseur(s) d'hydrocraquage est avantageusement comprise entre 0,5 et 15% poids et de préférence entre 1 et 10% poids, les pourcentages étant exprimés en pourcentage poids d'oxydes par rapport au poids total du catalyseur.

De préférence, la teneur en métal du groupe VIB dans le ou les catalyseur(s) d'hydrocraquage est avantageusement comprise entre 5 et 35% poids, et de préférence entre 10 et 30% poids, les pourcentages étant exprimés en pourcentage poids d'oxydes par rapport au poids total du catalyseur.

Le ou les catalyseur(s) d'hydrocraquage utilisés dans l'étape e') peuvent également comprendre éventuellement au moins un élément promoteur déposé sur le catalyseur et choisi dans le groupe formé par le phosphore, le bore et le silicium, éventuellement au moins un élément du groupe VIIA (chlore, fluor préférés), éventuellement au moins un élément du groupe VIIB (manganèse préféré), et éventuellement au moins un élément du groupe VB (niobium préféré).

De préférence, le ou les catalyseur(s) d'hydrocraquage utilisés dans l'étape e') comprennent au moins une matrice minérale poreuse amorphe ou mal cristallisée de type oxyde choisie parmi les alumines, les silices, les silice-alumines, les aluminates, l'alumine-oxyde de bore, la magnésie, la silice-magnésie, le zircone, l'oxyde de titane, l'argile, seuls ou en mélange, et de préférence les alumines ou les silice-alumines, seules ou en mélange.

De préférence, la silice-alumine contient plus de 50% poids d'alumine, de préférence plus de 60% poids d'alumine.

De préférence, le ou les catalyseur(s) d'hydrocraquage utilisés dans l'étape e') comprennent également éventuellement une zéolithe choisie parmi les zéolithes Y, de préférence parmi les zéolithes USY, seules ou en combinaison, avec d'autres zéolithes parmi les zéolithes beta, ZSM-12, IZM-2, ZSM-22, ZSM-23, SAPO-11, ZSM-48, ZBM-30, seules ou en mélange. De manière préférée la zéolithe est la zéolithe USY seule.

Dans le cas où ledit catalyseur comprend une zéolithe, la teneur en zéolithe dans le ou les catalyseur(s) d'hydrocraquage est avantageusement comprise entre 0,1 et 80% poids, de préférence comprise entre 3 et 70% poids, les pourcentages étant exprimés en pourcentage de zéolithe par rapport au poids total du catalyseur.

Un catalyseur préféré comprend, et est de préférence constitué, d'au moins un métal du groupe VIB et éventuellement d'au moins un métal du groupe VIII non noble, d'au moins un élément promoteur, et de préférence le phosphore, d'au moins une zéolithe Y et d'au moins un liant alumine.

Un catalyseur encore plus préféré comprend, et est de préférence constitué, du nickel, du molybdène, du phosphore, d'une zéolithe USY, et éventuellement aussi une zéolithe béta, et de l'alumine.

Un autre catalyseur préféré comprend, et est de préférence constitué, du nickel, du tungstène, de l'alumine et de la silice-alumine.

Un autre catalyseur préféré comprend, et est de préférence constitué, du nickel, du tungstène, d'une zéolithe USY, de l'alumine et de la silice-alumine.

Ledit catalyseur d'hydrocraquage est par exemple sous forme d'extrudés.

Selon un autre aspect de l'invention, le catalyseur d'hydrocraquage tel que décrit plus haut comprend en outre un ou plusieurs composés organiques contenant de l'oxygène et/ou de l'azote et/ou du soufre. Un tel catalyseur est souvent désigné par le terme "catalyseur additivé". Généralement, le composé organique est choisi parmi un composé comportant une ou plusieurs fonctions chimiques choisies parmi une fonction carboxylique, alcool, thiol, thioéther, sulfone, sulfoxyde, éther, aldéhyde, cétone, ester, carbonate, amine, nitrile, imide, oxime, urée et amide ou encore les composés incluant un cycle furanique ou encore les sucres.

**Etape f) de séparation**

Selon l'invention, le procédé de traitement comprend une étape f) de séparation, alimentée par l'effluent hydrotraité issu de l'étape e) ou par l'effluent hydrocraqué de l'étape e') optionnelle pour obtenir au moins un effluent gazeux et un effluent hydrocarboné liquide hydrotraité.

La section de séparation de l'étape f) est avantageusement réalisée dans des équipements de séparation bien connus (ballons séparateurs pouvant opérés à différentes pressions et températures, pompes, échangeurs de chaleurs, colonnes de stripage, colonne de distillation etc.).

L'effluent gazeux obtenu à l'issu de l'étape f) comprend avantageusement de l'hydrogène, de préférence comprend au moins 90% volume, de préférence au moins 95% volume, d'hydrogène. Avantageusement, ledit effluent gazeux peut au moins en partie être recyclé vers les étapes a) d'hydrogénation sélective et/ou b) d'hydroconversion et/ou e) d'hydrotraitement, et/ou e) d'hydrocraquage e') optionnel, le système de recyclage pouvant comprendre une section de purification.

L'effluent gazeux obtenu à l'issu de l'étape f) peut également comprendre des hydrocarbures légers, notamment de l'éthane, du propane et du butane, qui peuvent avantageusement être envoyés séparément ou en mélange dans un ou des fours de l'étape h) de vapocraquage.

L'étape f) permet en particulier d'éliminer les gaz dissous dans l'effluent liquide hydrocarboné, comme par exemple de l'ammoniac, de l'hydrogène sulfuré et des hydrocarbures légers ayant 1 à 4 atomes de carbone.

L'étape f) de fractionnement est avantageusement opérée à une pression inférieure ou égale à 1,0 MPa abs., de préférence entre 0,1 et 1,0 MPa abs.

Selon un mode de réalisation, l'étape f) peut être opérée dans une section comprenant avantageusement au moins une colonne de stripage équipée d'un circuit de reflux comprenant un ballon de reflux. Ladite colonne de stripage est alimentée par l'effluent hydrotraité issu de l'étape e) et par un flux de vapeur d'eau. L'effluent hydrotraité issu de l'étape e) peut être éventuellement réchauffé avant l'entrée dans la colonne de stripage. Ainsi, les composés les plus légers sont entrainés en tête de colonne et dans le circuit de reflux comprenant un ballon de reflux dans lequel s'opère une séparation gaz/liquide. La phase gazeuse qui comprend les hydrocarbures légers, est soutirée du ballon de reflux, en un flux gazeux. L'effluent hydrocarboné liquide hydrotraité qui est la coupe comprenant des composés ayant un point d'ébullition inférieur ou égal à 385°C hydrotraité (coupe naphta et coupe diesel) est avantageusement soutirée en fond de colonne de stripage.

Selon d'autres modes de réalisation, l'étape f) de séparation peut mettre en œuvre une colonne de stripage suivie d'une colonne de distillation ou uniquement une colonne de distillation.

Dans un mode de réalisation particulier, l'étape de séparation f) peut inclure un fractionnement permettant d'obtenir, outre un flux gazeux, une coupe naphta comprenant des composés ayant un point d'ébullition inférieur ou égal à 175°C, de préférence entre 80 et 175°C, et, une coupe diesel comprenant des composés ayant un point d'ébullition supérieur à 175°C et inférieur à 385°C.

La coupe naphta peut être envoyée, en tout ou partie, vers une unité de vapocraquage et/ou vers le pool naphta issu de charges pétrolières conventionnelles, elle peut encore être envoyée vers l'étape g) de recyclage.

La coupe diesel peut également être, en tout ou partie, soit envoyée vers une unité de vapocraquage, soit vers un pool carburéacteur et diesel issu de charges pétrolières conventionnelles, soit envoyée vers l'étape g) de recyclage, soit être introduite dans l'étape e') d'hydrocraquage lorsqu'elle est présente.

Dans un autre mode de réalisation particulier la coupe naphta comprenant des composés ayant un point d'ébullition inférieur ou égal à 175°C est fractionnée en une coupe naphta lourde comprenant des composés ayant un point d'ébullition entre 80 et 175°C et une coupe naphta légère comprenant des composés ayant un point d'ébullition inférieure à 80°C. Au moins une partie de ladite coupe naphta lourde peut être envoyée vers un complexe aromatique comportant au moins une étape de reformage du naphta en vue de produire des composés aromatiques. La coupe naphta lourde peut également être envoyée au moins en partie dans l'étape h) de vapocraquage décrite ci-dessous. Selon ce mode de réalisation, au moins une partie de la coupe naphta légère est envoyée dans l'étape h) de vapocraquage décrite ci-dessous.

**Etape g) (éventuelle) de recyclage de l'effluent hydrocarboné liquide hydrotraité issu de l'étape f)**

Le procédé selon l'invention peut comprendre l'étape g) de recyclage, dans laquelle une fraction de l'effluent hydrocarboné liquide hydrotraité issu de l'étape f) de séparation, est récupérée pour constituer un flux de recycle qui est envoyé en amont de ou directement vers au moins l'une des étapes réactionnelles du procédé selon l'invention, en particulier vers l'étape a) d'hydrogénation sélective optionnelle et/ou l'étape b) d'hydroconversion et/ou l'étape e) d'hydrotraitement. Eventuellement, une fraction du flux de recycle peut être envoyée vers l'étape a0) optionnelle de prétraitement.

De préférence, au moins une fraction de l'effluent hydrocarboné liquide hydrotraité issu de l'étape f) de séparation alimente l'étape e) d'hydrotraitement.

Avantageusement, la quantité du flux de recycle est ajustée de sorte que le rapport pondéral entre le flux de recycle et la charge comprenant une huile de pyrolyse, c'est-à-dire la charge à traiter alimentant le procédé global, est inférieur ou égal à 10, de préférence inférieur ou égal à 5, et préférentiellement supérieur ou égal à 0,001, de préférence supérieur ou égal à 0,01, et de manière préférée supérieur ou égal à 0,1. De manière très préférée, la quantité du flux de recycle est ajustée de sorte que le rapport pondéral entre le flux de recycle et la charge comprenant une huile de pyrolyse est compris entre 0,2 et 5.

Selon les charges traitées, le recyclage d'une partie du produit obtenu vers ou en amont de au moins une des étapes réactionnelles du procédé selon l'invention permet avantageusement d'une part de diluer les impuretés et d'autre part de contrôler la température dans la ou les étape(s) réactionnelle(s), dans la(les)quelle(s) des réactions mises en jeu peuvent être fortement exothermiques.

Selon un mode de réalisation préféré de l'invention, le procédé de traitement d'une charge comprenant une huile de pyrolyse comprend, de préférence consiste en, l'enchainement des étapes, et de préférence dans l'ordre donné :

b) d'hydroconversion,

c) de séparation,

d) de fractionnement

e) d'hydrotraitement,

f) de séparation,

pour produire un effluent dont au moins une partie est compatible pour un traitement dans une unité de vapocraquage.

Selon un deuxième mode de réalisation préféré de l'invention, le procédé de traitement d'une charge comprenant une huile de pyrolyse comprend, de préférence consiste en, l'enchainement des étapes, et de préférence dans l'ordre donné :

b) d'hydroconversion,

c) de séparation,

d) de fractionnement avec recyclage d'au moins une partie de la coupe hydrocarbonée comprenant des composés ayant un point d'ébullition supérieur à 385°C dans l'étape b),

e) d'hydrotraitement,

f) de séparation,

pour produire un effluent dont au moins une partie est compatible pour un traitement dans une unité de vapocraquage.

Selon un troisième mode de réalisation préféré de l'invention, le procédé de traitement d'une charge comprenant une huile de pyrolyse comprend, de préférence consiste en, l'enchainement des étapes, et de préférence dans l'ordre donné :

b) d'hydroconversion,

c) de séparation,

d) de fractionnement avec recyclage d'au moins une partie de la coupe hydrocarbonée comprenant des composés ayant un point d'ébullition supérieur à 385°C dans l'étape b),

e) d'hydrotraitement,

e') d'hydrocraquage,

f) de séparation,

pour produire un effluent dont au moins une partie est compatible pour un traitement dans une unité de vapocraquage.

Selon un quatrième mode de réalisation préféré de l'invention, le procédé de traitement d'une charge comprenant une huile de pyrolyse de plastiques et/ou de CSR comprend, de préférence consiste en, l'enchainement des étapes, et de préférence dans l'ordre donné :

a) d'hydrogénation sélective,

b) d'hydroconversion,

c) de séparation,

d) de fractionnement avec recyclage d'au moins une partie de la coupe hydrocarbonée comprenant des composés ayant un point d'ébullition supérieur à 385°C dans l'étape b),

e) d'hydrotraitement,

e') d'hydrocraquage,

f) de séparation,

g) de recyclage d'au moins une partie de l'effluent hydrocarboné liquide hydrotraité issue de l'étape f) aux étapes a) et/ou b) et/ou e),

pour produire un effluent dont au moins une partie est compatible pour un traitement dans une unité de vapocraquage.

Ledit effluent hydrocarboné liquide hydrotraité ou une fraction dudit effluent obtenu par fractionnement ainsi obtenu(e)(s) par traitement selon le procédé de l'invention d'une huile de pyrolyse, présente(nt) une composition compatible avec les spécifications d'une charge en entrée d'une unité de vapocraquage. En particulier, la composition de l'effluent hydrocarboné ou du(des)dit(s) flux hydrocarboné(s) est de préférence telle que :

- la teneur totale en éléments métalliques est inférieure ou égale à 5,0 ppm poids, de préférence inférieure ou égale à 2,0 ppm poids, préférentiellement inférieure ou égale à 1,0 ppm poids et de manière préférée inférieure ou égale à 0,5 ppm poids, avec :
une teneur en élément fer (Fe) inférieure ou égale à 100 ppb poids, et
- la teneur en élément silicium (Si) inférieure ou égale à 1,0 ppm poids, de préférence inférieure ou égale à 0,6 ppm poids, et

- la teneur en soufre est inférieure ou égale à 500 ppm poids, de préférence inférieure ou égale à 200 ppm poids,
- la teneur en azote est inférieure ou égale à 100 ppm poids, de préférence inférieure ou égale à 50 ppm poids et de manière préférée inférieure ou égale à 5 ppm poids,
- la teneur totale en élément chlore est inférieure ou égale à 10 ppm poids, de manière préférée inférieure à 1,0 ppm poids,
- la teneur en asphaltènes est inférieure ou égale à 5,0 ppm poids,
- la teneur en composés oléfiniques (mono- et di-oléfines) est inférieure ou égale à 5,0% poids, de préférence inférieure ou égale à 2,0% poids, de manière préférée inférieure ou égale à 0,1% poids.

Les teneurs sont données en concentrations pondérales relatives, pourcentage (%) poids, partie(s) par million (ppm) poids ou partie(s) par milliard (ppb) poids, par rapport au poids total du flux considéré.

Le procédé selon l'invention permet donc de traiter les huiles de pyrolyse de plastiques et/ou de CSR pour obtenir un effluent qui peut être injecté, en tout ou partie, dans une unité de vapocraquage.

**Etape h) de vapocraquage (optionnelle)**

L'effluent hydrocarboné liquide hydrotraité issu de l'étape f), tout ou partie, peut être envoyée vers une étape h) de vapocraquage.

De manière avantageuse, la ou les fractions gaz issue(s) de l'étape c) et/ou f) de séparation et contenant de l'éthane, du propane et du butane, peut (peuvent) en tout ou partie être également envoyé vers l'étape h) de vapocraquage.

Ladite étape h) de vapocraquage est avantageusement réalisée dans au moins un four de pyrolyse à une température comprise entre 700 et 900°C, de préférence entre 750 et 850°C, et à une pression comprise entre 0,05 et 0,3 MPa relatif. Le temps de séjour des composés hydrocarbonés est généralement inférieur ou égale à 1,0 seconde (noté s), de préférence compris entre 0,1 et 0,5 s. Avantageusement, de la vapeur d'eau est introduite en amont de l'étape h) de vapocraquage optionnelle et après la séparation (ou le fractionnement). La quantité d'eau introduite, avantageusement sous forme de vapeur d'eau, est avantageusement comprise entre 0,3 et 3,0 kg d'eau par kg de composés hydrocarbonés en entrée de l'étape h). De préférence, l'étape h) optionnelle est réalisée dans plusieurs fours de pyrolyse en parallèle de manière à adapter les conditions opératoires aux différents flux alimentant l'étape h) notamment issus des étapes c) et/ou f), et aussi à gérer les temps de décokage des tubes. Un four comprend un ou plusieurs tubes disposés en parallèle. Un four peut également désigner un groupe de fours opérant en parallèle. Par exemple, un four peut être dédié au craquage de la coupe naphta comprenant des composés ayant une température d'ébullition inférieure ou égal à 175°C.

Les effluents des différents fours de vapocraquage sont généralement recombinés avant séparation en vue de constituer un effluent. Il est entendu que l'étape h) de vapocraquage comporte les fours de vapocraquage mais aussi les sous étapes associées au vapocraquage bien connues de l'Homme du métier. Ces sous étapes peuvent comporter notamment des échangeurs de chaleur, des colonnes et des réacteurs catalytiques et des recyclages vers les fours. Une colonne permet généralement de fractionner l'effluent en vue de récupérer au moins une fraction légère comprenant de l'hydrogène et des composés ayant 2 à 5 atomes de carbone, et une fraction comprenant de l'essence de pyrolyse, et éventuellement une fraction comprenant de l'huile de pyrolyse. Des colonnes permettent de séparer les différents constituants de la fraction légère de fractionnement afin de récupérer au moins une coupe riche en éthylène (coupe C2) et une coupe riche en propylène (coupe C3) et éventuellement une coupe riche en butènes (coupe C4). Les réacteurs catalytiques permettent notamment de réaliser des hydrogénations sélectives des coupes C2, C3 voire C4 et de l'essence de pyrolyse. Les composés saturés, notamment les composés saturés ayant 2 à 4 atomes de carbone sont avantageusement recyclés vers les fours de vapocraquage de manière à accroitre les rendements globaux en oléfines.

Cette étape h) de vapocraquage permet d'obtenir au moins un effluent contenant des oléfines comprenant 2, 3 et/ou 4 atomes de carbone (c'est-à-dire des oléfines en C2, C3 et/ou C4), à des teneurs satisfaisantes, en particulier supérieures ou égales à 30% poids, notamment supérieures ou égales 40% poids, voire supérieures ou égales 50% poids d'oléfines totales comprenant 2, 3 et 4 atomes de carbone par rapport au poids de l'effluent de vapocraquage considéré. Lesdites oléfines en C2, C3 et C4 peuvent ensuite être avantageusement utilisées comme monomères de polyoléfines.

Selon un ou plusieurs mode(s) de réalisation préféré(s) de l'invention, pris séparément ou combinés entre eux, le procédé de traitement d'une charge comprenant une huile de pyrolyse de plastiques et/ou de CSR comprend, de préférence consiste en, l'enchainement des étapes décrites ci-dessus, et de préférence dans l'ordre donné :

b) d'hydroconversion,
c) de séparation,

d) de fractionnement

e) d'hydrotraitement,

f) de séparation,

h) de vapocraquage.

Selon un mode préféré, le procédé de traitement d'une charge comprenant une huile de pyrolyse de plastiques et/ou de CSR comprend, de préférence consiste en, l'enchainement des étapes décrites ci-dessus, et de préférence dans l'ordre donné :

a) d'hydrogénation sélective,

b) d'hydroconversion,

c) de séparation,

d) de fractionnement avec recyclage d'au moins une partie de la coupe hydrocarbonée comprenant des composés ayant un point d'ébullition supérieur à 385°C dans l'étape b),

e) d'hydrotraitement,

e') d'hydrocraquage,

f) de séparation,

h) de vapocraquage d'au moins l'autre partie de l'effluent hydrocarboné liquide hydrotraité issue de l'étape f)

**Méthodes d'analyse utilisées**

Les méthodes d'analyses et/ou normes utilisées pour déterminer les caractéristiques des différents flux en particuliers de la charge à traiter et des effluents, sont connues de l'Homme du métier. Elles sont en particulier listées ci-dessous à titre de renseignements. D'autres méthodes réputées équivalentes peuvent aussi être utilisées, notamment des méthodes équivalentes IP, EN ou ISO :

*Tableau 1*

| Description | Méthodes |
|---|---|
| Masse volumique @15°C | ASTM D4052 |
| Teneur en Soufre | ISO 20846 |
| Teneur en Azote | ASTM D4629 |
| Indice d'acide | ASTM D664 |
| Nombre de Brome | ASTM D1159 |
| Teneur en Di-oléfines à partir de l'indice d'anhydride Maléique | Méthode MAV (1) |
| Teneur en Oxygénés | Combustion + Infrarouqe |
| Teneur en Paraffines | UOP990-11 |
| Teneur en Naphthènes et Oléfines | UOP990-11 |
| Teneur en Aromatiques | UOP990-11 |
| Teneur en Halogènes | ASTM D7359 |
| Teneur en Chlorure | ASTM D7536 |
| Teneur en Métaux : | ASTM D5185 |
| P | |
| Fe | |
| Si | |
| Na | |
| B | |

(suite)

| Description | Méthodes |
|---|---|
| Distillation simulée | ASTM D2887 |

| (1) Méthode MAV décrite dans l'article : C. López-García et al., Near Infrared Monitoring of Low Conjugated Diolefins Content in Hydrotreated FCC Gasoline Streams, Oil & Gas Science and Technology - Rev. IFP, Vol. 62 (2007), No. 1, pp. 57-68 |
|---|

**LISTE DES FIGURES**

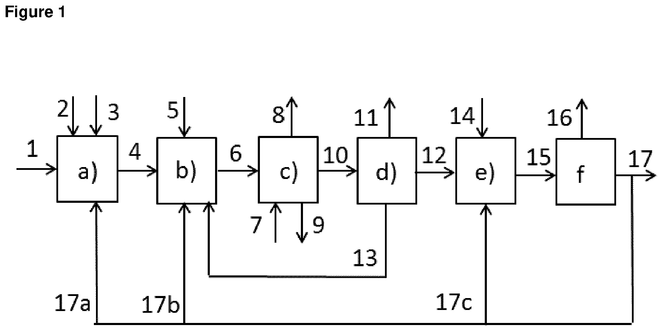

La mention des éléments référencés dans la Figure 1 permet une meilleure compréhension de l'invention, sans que celle-ci ne se limite aux modes de réalisation particuliers illustrés dans la Figure 1. Les différents modes de réalisation présentés peuvent être utilisés seuls ou en combinaison les uns avec les autres, sans limitation de combinaison.

La Figure 1 représente le schéma d'un mode de réalisation particulier du procédé de la présente invention, comprenant :

- une étape a) d'hydrogénation sélective optionnelle d'une charge hydrocarbonée issue de la pyrolyse 1, en présence d'un gaz riche en hydrogène 2 et éventuellement d'une amine apportée par le flux 3, réalisée dans au moins un réacteur en lit fixe comportant au moins un catalyseur d'hydrogénation sélective, pour obtenir un effluent 4 ;

- une étape b) d'hydroconversion de l'effluent 4 issu de l'étape a), en présence d'hydrogène 5 réalisée dans au moins un réacteur en lit bouillonnant, à lit entraîné et/ou à lit mobile comportant au moins un catalyseur d'hydroconversion, pour obtenir un effluent hydroconverti 6 ;

- une étape c) de séparation de l'effluent 6 réalisée en présence d'une solution aqueuse de lavage 7 et permettant d'obtenir au moins une fraction 8 comprenant de l'hydrogène, une fraction aqueuse 9 contenant des sels dissous, et une fraction liquide hydrocarbonée 10 ;

- une étape d) de fractionnement de la fraction liquide hydrocarbonée 10 permettant d'obtenir au moins une fraction gazeuse 11, une coupe hydrocarbonée 12 comprenant des composés ayant un point d'ébullition inférieur ou égal à 385°C et une coupe hydrocarbonée 13 comprenant des composés ayant un point d'ébullition supérieur à 385°C qui est de préférence au moins en partie recyclée dans l'étape b) ;

- une étape e) d'hydrotraitement d'au moins une partie de la coupe hydrocarbonée 12 comprenant des composés ayant un point d'ébullition inférieur ou égal à 385°C issu de l'étape d), en présence d'hydrogène 14 réalisée dans au moins un réacteur en lit fixe comportant au moins un catalyseur d'hydrotraitement, pour obtenir un effluent hydrotraité 15 ;

- une étape f) de séparation de l'effluent 15 permettant d'obtenir au moins une fraction 16 comprenant de l'hydrogène et un effluent hydrocarboné liquide hydrotraité 17.

A l'issue de l'étape f), au moins une partie de l'effluent hydrocarboné liquide hydrotraité 17 est envoyée(s) vers un procédé de vapocraquage (non représentée).

Optionnellement, une partie dudit effluent hydrocarboné liquide hydrotraité 17 constitue un flux de recycle 17a, 17b et 17c qui alimente les étapes a) et/ou b) et/ou e) respectivement.

Seules les principales étapes, avec les flux principaux, sont représentées sur la Figure 1, afin de permettre une meilleure compréhension de l'invention. Il est bien entendu que tous les équipements nécessaires au fonctionnement sont présents (ballons, pompes, échangeurs, fours, colonnes, etc.), même si non représentés. Il est également entendu que des flux de gaz riche en hydrogène (appoint ou recycle), comme décrit ci-dessus, peuvent être injectés en entrée de chaque réacteur ou lit catalytique ou entre deux réacteurs ou deux lits catalytiques. Des moyens bien connus de l'homme du métier de purification et de recyclage d'hydrogène peuvent être également mis en œuvre.

**EXEMPLES**

**Exemple 1 (conforme à l'invention)**

La charge 1 traitée dans le procédé est une huile de pyrolyse de CSR présentant les caractéristiques indiquées dans le tableau 2.

*Tableau 2 : caractéristiques de la charge*

| Description | Méthodes | Unité | Huile de pyrolyse |
|---|---|---|---|
| Masse volumique @15°C | ASTM D4052 | g/cm$^3$ | 0,91 |
| Teneur en Soufre | ISO 20846 | ppm poids | 3500 |
| Teneur en Azote | ASTM D4629 | ppm poids | 2900 |
| Indice d'acide | ASTM D664 | mg KOH/g | 15 |
| Nombre de Brome | ASTM D1159 | g/100g | 60 |
| Teneur en Di-oléfines à partir de l'indice d'anhydride Maléique | Méthode MAV | % poids | 7,0 |
| Teneur en Oxygénés | Combustion + Infra-rouge | % poids | 1,0 |
| Teneur en Paraffines | UOP990-11 | % poids | 15 |
| Teneur en Naphtènes et Oléfines | UOP990-11 | % poids | 25 |
| Teneur en Aromatiques | UOP990-11 | % poids | 60 |
| Teneur en Halogènes | ASTM D7359 | ppm poids | 400 |
| Teneur en Chlorure | ASTM D7536 | ppm poids | 300 |
| Teneur en Métaux : | ASTM D5185 | | |
| P | | ppm poids | 20 |
| Fe | | ppm poids | 30 |
| Si | | ppm poids | 500 |
| Na | | ppm poids | 2 |
| B | | ppm poids | 2 |
| Distillation simulée | ASTM D2887 | | |
| 0% | | °C | 50 |
| 10% | | °C | 120 |
| 30% | | °C | 145 |
| 50% | | °C | 170 |
| 70% | | °C | 230 |
| 90% | | °C | 320 |
| 100% | | °C | 405 |

La charge 1 est soumise directement (sans étape a) d'hydrogénation sélective) à une étape b) d'hydroconversion réalisée en lit bouillonnant et en présence d'hydrogène 5, et d'un catalyseur d'hydrotraitement de type NiMo sur alumine dans les conditions présentées dans le tableau 3.

*Tableau 3 : conditions de l'étape b) d'hydroconversion*

| Température d'hydroconversion | °C | 400 |
|---|---|---|
| Pression Partielle d'Hydrogène | MPa abs | 9,0 |
| H$_2$/HC (Couverture volumique d'hydrogène par rapport au volume de charge) | Nm$^3$/m$^3$ | 300 |
| VVH (débit volumique de charge/volume de catalyseurs) | h-1 | 1,0 |

L'effluent 6 issu de l'étape b) d'hydroconversion est envoyé à l'étape c) de séparation et ensuite à l'étape d) de fractionnement. Le tableau 4 donne les rendements des différentes fractions obtenues en sortie de l'étape d) de fractionnement par rapport à la charge 1 en entrée de la chaine de procédé.

*Tableau 4 : rendements des différents produits et fractions obtenus en sortie de l'étape d)*

| H$_2$S + NH$_3$ | % poids | 0,35 |
|---|---|---|
| C1-C4 | % poids | 3,70 |
| Fraction PI-175°C | % poids | 59,0 |
| Fraction 175°C-385°C | % poids | 35,0 |
| Fraction 385°C+ | % poids | 3,0 |
| Total | % poids | 101,05 |

La coupe liquide comprenant des composés ayant un point d'ébullition inférieur ou égal à 385°C (coupe naphta et coupe diesel) est ensuite envoyée vers l'étape e) d'hydrotraitement et en présence d'hydrogène et d'un catalyseur d'hydrotraitement de type NiMo sur alumine dans les conditions présentées dans le tableau 5.

*Tableau 5 : conditions de l'étape e) d'hydrotraitement*

| Température d'hydrotraitement | °C | 350 |
|---|---|---|
| Pression Partielle d'Hydrogène | MPa abs | 9,0 |
| H$_2$/HC (Couverture volumique d'hydrogène par rapport au volume de charge) | Nm$^3$/m$^3$ | 300 |
| VVH (débit volumique de charge/volume de catalyseurs) | h-1 | 0,5 |

L'effluent 17 issu de l'étape e) d'hydrotraitement est soumis à une étape f) de séparation et de fractionnement.
Le tableau 6 donne les rendements globaux par rapport à la charge 1 en entrée de la chaine de procédé des différentes fractions obtenues en sortie de l'étape f) de séparation et de fractionnement (qui comprend une colonne de stripage et une colonne de distillation).

*Tableau 6 : rendements des différents produits et fractions obtenus en sortie de l'étape f)*

| H$_2$S + NH$_3$ | % poids | 0,50 |
|---|---|---|
| C1-C4 | % poids | 4,50 |
| Fraction PI-175°C | % poids | 60,5 |
| Fraction 175°C-385°C | % poids | 32,5 |
| Fraction 385°C+ | % poids | 2,5 |
| Total | % poids | 100,50 |

Les composés H$_2$S et NH$_3$ sont principalement éliminés sous forme de sels dans la phase aqueuse éliminée à l'étape d) de séparation.
Les caractéristiques des fractions liquides PI-175°C et 175°C-385°C obtenues après l'étape f) de séparation et de fractionnement sont présentés tableau 7 :

*Tableau 7: caractéristiques des fraction PI-175°C, 175°C-385°C*

| | Unité | Fraction PI-175°C | Fraction 175°C-385°C |
|---|---|---|---|
| Masse volumique @ 15°C (ASTM D4052) | g/cm$^3$ | 0,762 | 0,813 |
| Teneur en : | | | |
| Soufre (ASTM D5453) | ppm poids | < 2 | < 2 |
| Azote (ASTM D4629) | ppm poids | < 5 | < 10 |
| Fe (ASTM D5185) | ppb poids | Non détecté | 25 |
| Métaux Totaux (ASTM D5185) | ppm poids | Non détecté | 1 |
| Chlore (ASTM D7536) | ppb poids | Non détecté | < 25 |
| Paraffines (UOP990-11) | % poids | 45 | 30 |
| Naphtènes (UOP990-11) | % poids | 50 | 60 |
| Oléfines (UOP990-11) | % poids | 5 | 10 |
| Aromatiques (UOP990-11) | % poids | 1 | 2 |
| Distillation Simulée (ASTM D2887) en % | | | |
| 0 | °C | 25 | 175 |
| 5 | °C | 38 | 185 |
| 10 | °C | 59 | 210 |
| 30 | °C | 90 | 240 |
| 50 | °C | 118 | 275 |
| 70 | °C | 140 | 310 |
| 90 | °C | 163 | 360 |
| 95 | °C | 175 | 385 |
| 100 | °C | 180 | 395 |

Les fractions liquides PI-175°C et 175°C-385°C présentent toutes les deux des compositions compatibles avec une unité de vapocraquage puisque :

- elles présentent des teneurs en élément chlore très faibles (respectivement une teneur non détectée et une teneur de 25 ppb poids) et inférieures à la limite requise pour une charge de vapocraqueur ;
- les teneurs en métaux, en particulier en fer (Fe), sont elles aussi très faibles (teneurs en métaux non détectée pour la fraction PI-175°C et < 1 ppm poids pour la fraction 175°C-385°C ; teneurs en Fe non détectée pour la fraction PI-175°C et 25 ppb poids pour la fraction 175°C-385°C) et inférieures aux limites requises pour une charge de vapocraqueur ($\leq$ 5,0 ppm poids, de manière très préférée $\leq$ 1 ppm poids pour les métaux ; $\leq$ 100 ppb poids pour le Fe) ;
- enfin elles contiennent du soufre (< 2 ppm poids pour la fraction PI-175°C et < 2 ppm poids pour la fraction 175°C-385°C) et de l'azote (< 5 ppm poids pour la fraction PI-175°C et < 10ppm poids pour la fraction 175°C-385°C) à des teneurs très inférieures aux limites requises pour une charge de vapocraqueur ($\leq$ 500 ppm poids, de préférence $\leq$ 200 ppm poids pour S et N).

Les fractions liquides PI-175°C et 175°C-385°C obtenues sont donc ensuite envoyées vers une étape h) de vapocraquage (cf. tableau 8).

*Tableau 8 : conditions de l'étape de vapocraquage*

| | Unité | Fractions PI-175°C | Fraction 175-385°C+ |
|---|---|---|---|
| Pression sortie fours | MPa abs | 0,2 | 0,2 |
| Température sortie four | °C | 835 | 820 |

(suite)

| | Unité | Fractions PI-175°C | Fraction 175-385°C+ |
|---|---|---|---|
| Ratio Vapeur/charge | kg/kg | 0,6 | 0,8 |
| Temps de séjour four | s | 0,25 | 0,2 |

Les effluents des différents fours de vapocraquage sont soumis à une étape de séparation permettant de recycler les composés saturés vers les fours de vapocraquage et d'obtenir les rendements présentés dans le tableau 9 (rendement = % de masse de produit par rapport à la masse de la fraction PI-175°C et 175°C-385°C en amont de l'étape de vapocraquage, noté % poids).

*Tableau 9 : rendements de l'étape de vapocraquage*

| Fraction | | Fraction PI-175°C | Fraction 175°C-385°C |
|---|---|---|---|
| $H_2$, CO, C1 | % poids | 19,0 | 12,0 |
| Ethylène | % poids | 33,0 | 26,0 |
| Propylène | % poids | 16,0 | 16,0 |
| Coupe C4 | % poids | 9,0 | 9,0 |
| Essence de pyrolyse | % poids | 17,0 | 19,0 |
| Huile de pyrolyse | % poids | 6,0 | 18,0 |

En considérant les rendements obtenus pour les fractions liquides PI-175°C et 175°C-385°C lors du procédé de traitement de l'huile de pyrolyse en sorti des étapes d'hydroconversion et d'hydrotraitement (cf. tableau 6), il est possible de déterminer les rendements globaux par rapport à la charge 1 en entrée de la chaine de procédé des produits issus de l'étape h) de vapocraquage par rapport à la charge initiale de type huile de pyrolyse de CSR introduite à l'étape a) :

*Tableau 10 : rendements en produits issus de l'étape de vapocraquage de la fraction PI-175°C et de la fraction 175°C-385°C*

| | | |
|---|---|---|
| $H_2S + NH_3$ | % poids | 0,50 |
| H2, CO, C1 | % poids | 16,0 |
| C2 | % poids | 0,6 |
| C3 | % poids | 1,7 |
| C4 | % poids | 1,8 |
| Ethylène | % poids | 28,7 |
| Propylène | % poids | 15,1 |
| Coupe C4 | % poids | 8,4 |
| Essence de pyrolyse | % poids | 16,7 |
| Huile de pyrolyse | % poids | 9,5 |
| Fraction 385°C+ | % poids | 2,5 |
| Total | % poids | 101,50 |

Lorsque les fractions PI-175°C et 175-385°C sont envoyées à l'unité de vapocraquage, le procédé selon l'invention permet d'atteindre des rendements massiques globaux en éthylène et en propylène respectivement de 28,7 % et 15,1% par rapport à la quantité massique de charge de type huile de pyrolyse initiale.

De plus, l'enchainement spécifique d'étapes en amont de l'étape de vapocraquage permet de limiter la formation de coke et d'éviter les problèmes de corrosion qui seraient apparus si le chlore n'avait pas été éliminé.

**Exemple 2 (conforme à l'invention)**

La charge 1 traitée dans le procédé est une huile de pyrolyse de plastiques présentant les caractéristiques indiquées dans le tableau 11.

*Tableau 11 : caractéristiques de la charge*

| Description | Méthodes | Unité | Huile de pyrolyse |
|---|---|---|---|
| Masse volumique @15°C | ASTM D4052 | g/cm$^3$ | 0,82 |
| Teneur en Soufre | ISO 20846 | ppm poids | 2500 |
| Teneur en Azote | ASTM D4629 | ppm poids | 730 |
| Indice d'acide | ASTM D664 | mg KOH/g | 1,5 |
| Nombre de Brome | ASTM D1159 | g/100g | 80 |
| Teneur en Di-oléfines à partir de l'indice d'anhydride Maléique | Méthode MAV | % poids | 10,0 |
| Teneur en Oxygénés | Combustion + Infra-rouqe | % poids | 1,0 |
| Teneur en Paraffines | UOP990-11 | % poids | 45 |
| Teneur en Naphtènes et Oléfines | UOP990-11 | % poids | 45 |
| Teneur en Aromatiques | UOP990-11 | % poids | 10 |
| Teneur en Halogènes | ASTM D7359 | ppm poids | 350 |
| Teneur en Chlorure | ASTM D7536 | ppm poids | 320 |
| Teneur en Métaux : | ASTM D5185 | | |
| P | | ppm poids | 10 |
| Fe | | ppm poids | 25 |
| Si | | ppm poids | 45 |
| Na | | ppm poids | 2 |
| B | | ppm poids | 2 |
| Distillation simulée | ASTM D2887 | | |
| 0% | | °C | 40 |
| 10% | | °C | 98 |
| 30% | | °C | 161 |
| 50% | | °C | 232 |
| 70% | | °C | 309 |
| 90% | | °C | 394 |
| 100% | | °C | 432 |

La charge 1 est soumise à une étape a) d'hydrogénation sélective réalisée dans un réacteur en lit fixe et en présence d'hydrogène 2 et d'un catalyseur d'hydrogénation sélective de type NiMo sur alumine dans les conditions indiquées dans le tableau 12.

*Tableau 12 : conditions de l'étape a) d'hydrogénation sélective*

| Température | °C | 180 |
|---|---|---|
| Pression Partielle d'Hydrogène | MPa abs | 9,0 |

(suite)

| Température | °C | 180 |
| --- | --- | --- |
| $H_2$/HC (Couverture volumique d'hydrogène par rapport au volume de charge) | $Nm^3/m^3$ | 50 |
| VVH (débit volumique de charge/volume de catalyseurs) | $h^{-1}$ | 0,5 |

A l'issue de l'étape a) d'hydrogénation sélective, la teneur en dioléfines dans la charge a été significativement réduite.

L'effluent 4 issu de l'étape a) d'hydrogénation sélective est soumis directement, sans séparation, à une étape b) d'hydroconversion réalisée en lit bouillonnant et en présence d'hydrogène 5, et d'un catalyseur d'hydrotraitement de type NiMo sur alumine dans les conditions présentées dans le tableau 13.

*Tableau 13 : conditions de l'étape b) d'hydroconversion*

| Température d'hydroconversion | °C | 380 |
| --- | --- | --- |
| Pression Partielle d'Hydrogène | MPa abs | 9,0 |
| $H_2$/HC (Couverture volumique d'hydrogène par rapport au volume de charge) | $Nm^3/m^3$ | 300 |
| VVH (débit volumique de charge étape b)/volume de catalyseurs) | $h^{-1}$ | 1,5 |

L'effluent 6 issu de l'étape b) d'hydroconversion est envoyé à l'étape c) de séparation et ensuite à l'étape d) de fractionnement. Le tableau 14 donne les rendements des différentes fractions obtenues en sortie de l'étape d) de fractionnement, par rapport à la charge 1 en entrée de la chaine de procédé.

*Tableau 14 : rendements des différents produits et fractions obtenus en sortie de l'étape d) de fractionnement*

| $H_2S$+ $NH_3$ | % poids | 0,4 |
| --- | --- | --- |
| C1-C4 | % poids | 1,0 |
| Fraction PI-150°C | % poids | 28,4 |
| Fraction 150°C+ | % poids | 70,9 |
| Total | % poids | 100,7 |

La coupe liquide comprenant des composés ayant un point d'ébullition inférieur ou égal à 385°C (coupe naphta PI-150°C et coupe diesel 150°C+) est ensuite envoyée vers l'étape e) d'hydrotraitement et en présence d'hydrogène et d'un catalyseur d'hydrotraitement de type NiMo sur alumine dans les conditions présentées dans le tableau 15.

*Tableau 15 : conditions de l'étape e) d'hydrotraitement*

| Température d'hydrotraitement | °C | 350 |
| --- | --- | --- |
| Pression Partielle d'Hydrogène | MPa abs | 9,0 |
| $H_2$/HC (Couverture volumique d'hydrogène par rapport au volume de charge) | $Nm^3/m^3$ | 300 |
| VVH (débit volumique de charge/volume de catalyseurs) | $h^{-1}$ | 1 |

L'effluent 17 issu de l'étape e) d'hydrotraitement est soumis à une étape f) de séparation et de fractionnement.

Le tableau 16 donne les rendements globaux, par rapport à la charge 1 en entrée de la chaine de procédé, des différentes fractions obtenues en sortie de l'étape f) de séparation et de fractionnement (qui comprend une colonne de stripage et une colonne de distillation).

*Tableau 16 : rendements des différents produits et fractions obtenus en sortie de l'étape f) de séparation et de fractionnement*

| $H_2S$+ $NH_3$ | % poids | 0,5 |
| --- | --- | --- |

(suite)

| C1-C4 | % poids | 1,1 |
| Fraction PI-150°C | % poids | 28,6 |
| Fraction 150°C+ | % poids | 70,7 |
| Total | % poids | 100,9 |

Les composés $H_2S$ et $NH_3$ sont principalement éliminés sous forme de sels dans la phase aqueuse éliminée à l'étape d) de séparation.

Les caractéristiques des fractions liquides PI-150°C et 150°C+ obtenues après l'étape f) de séparation et de fractionnement sont présentés tableau 17 :

*Tableau 17 : caractéristiques des fraction PI-150°C et 150°C+ après l'étape f) de séparation et de fractionnement*

| | | Fraction PI-150°C | Fraction 150 °C+ |
| --- | --- | --- | --- |
| Masse volumique @ 15°C (ASTM D4052) | g/cm$^3$ | 0,750 | 0,820 |
| Teneur en : | | | |
| Soufre (ASTM D5453) | ppm poids | <2 | <2 |
| Azote (ASTM D4629) | ppm poids | < 5 | < 10 |
| Fe (ASTM D5185) | ppb poids | Non détecté | <50 |
| Métaux Totaux (ASTM D5185) | ppm poids | Non détecté | <1 |
| Chlore (ASTM D7536) | ppb poids | Non détecté | < 25 |
| Paraffines (UOP990-11) | % poids | 75 | 70 |
| Naphtènes (UOP990-11) | % poids | 25 | 28 |
| Oléfines (UOP990-11) | % poids | Non détecté | Non détecté |
| Aromatiques (UOP990-11) | % poids | <1 | 2 |
| Distillation Simulée (ASTM D2887) en % | | | |
| 0 | °C | 25 | 140 |
| 5 | °C | 32 | 162 |
| 10 | °C | 40 | 174 |
| 30 | °C | 82 | 226 |
| 50 | °C | 108 | 281 |
| 70 | °C | 126 | 346 |
| 90 | °C | 142 | 395 |
| 95 | °C | 146 | 404 |
| 100 | °C | 160 | 425 |

Les fractions liquides PI-150°C et 150°C+ présentent toutes les deux des compositions compatibles avec une unité de vapocraquage puisque :

- elles ne contiennent pas d'oléfines (mono- et di-oléfines) ;
- elles présentent des teneurs en élément chlore très faibles (respectivement une teneur non détectée et une teneur de 25 ppb poids) et inférieures à la limite requise pour une charge de vapocraqueur ;
- les teneurs en métaux, en particulier en fer (Fe), sont elles aussi très faibles (teneurs en métaux non détectée pour la fraction PI-150°C et < 1 ppm poids pour la fraction 150°C+; teneurs en Fe non détectée pour la fraction PI-150°C et < 50 ppb poids pour la fraction 150°C+) et inférieures aux limites requises pour une charge de vapocraqueur ($\leq$

5,0 ppm poids, de manière très préférée ≤ 1 ppm poids pour les métaux ; ≤ 100 ppb poids pour le Fe) ;
- enfin elles contiennent du soufre (< 2 ppm poids pour la fraction PI-150°C et < 2 ppm poids pour la fraction 150°C+ et de l'azote (< 5 ppm poids pour la fraction PI-150°C et < 10ppm poids pour la fraction 150°C+°C) à des teneurs très inférieures aux limites requises pour une charge de vapocraqueur (≤ 500 ppm poids, de préférence ≤ 200 ppm poids pour S et N).

Les fractions liquides PI-150°C et 150°C+ obtenues sont avantageusement ensuite envoyées vers une étape h) de vapocraquage.

**Revendications**

1. Procédé de traitement d'une charge comprenant une huile de pyrolyse de plastiques et/ou de combustibles solides de récupération, comprenant :

   a) optionnellement, une étape d'hydrogénation sélective mise en œuvre dans une section réactionnelle alimentée au moins par ladite charge et un flux gazeux comprenant de l'hydrogène, en présence d'au moins un catalyseur d'hydrogénation sélective, à une température entre 100 et 280°C, une pression partielle d'hydrogène entre 1,0 et 20,0 MPa abs. et une vitesse volumique horaire entre 0,3 et 10,0 $h^{-1}$, pour obtenir un effluent hydrogéné ;
   b) une étape d'hydroconversion mise en œuvre dans une section réactionnelle d'hydroconversion, mettant en œuvre au moins un réacteur à lit bouillonnant, à lit entraîné et/ou à lit mobile, comprenant au moins un catalyseur d'hydroconversion, ladite section réactionnelle d'hydroconversion étant alimentée au moins par ladite charge ou par ledit effluent hydrogéné issu de l'étape a) et un flux gazeux comprenant de l'hydrogène, ladite section réactionnelle d'hydroconversion étant mise en œuvre à une température entre 250 et 450°C, une pression partielle d'hydrogène entre 1,0 et 20,0 MPa abs et une vitesse volumique horaire entre 0,05 et 10,0 $h^{-1}$, pour obtenir un effluent d'hydroconverti ;
   c) une étape de séparation, alimentée par l'effluent hydroconverti issu de l'étape b) et une solution aqueuse, ladite étape étant opérée à une température entre 50 et 450°C, pour obtenir au moins un effluent gazeux, un effluent aqueux et un effluent hydrocarboné ;
   d) une étape de fractionnement de tout ou partie de l'effluent hydrocarboné issu de l'étape c), pour obtenir au moins un flux gazeux, une coupe hydrocarbonée comprenant des composés ayant un point d'ébullition inférieur ou égal à 385°C et une coupe hydrocarbonée comprenant des composés ayant un point d'ébullition supérieur à 385°C,
   e) une étape d'hydrotraitement mise en œuvre dans une section réactionnelle d'hydrotraitement, mettant en œuvre au moins un réacteur à lit fixe ayant n lits catalytiques, n étant un nombre entier supérieur ou égal à 1, comprenant chacun au moins un catalyseur d'hydrotraitement, ladite section réactionnelle d'hydrotraitement étant alimentée par au moins une partie de ladite coupe hydrocarbonée comprenant des composés ayant un point d'ébullition inférieur ou égal à 385°C issue de l'étape d) et un flux gazeux comprenant de l'hydrogène, ladite section réactionnelle d'hydrotraitement étant mise en œuvre à une température entre 250 et 430°C, une pression partielle d'hydrogène entre 1,0 et 20,0 MPa abs. et une vitesse volumique horaire entre 0,1 et 10,0 $h^{-1}$, pour obtenir un effluent hydrotraité ;
   f) une étape de séparation, alimentée par l'effluent hydrotraité issu de l'étape e) pour obtenir au moins un effluent gazeux et un effluent hydrocarboné liquide hydrotraité.

2. Procédé selon la revendication précédente comprenant ladite étape a) d'hydrogénation sélective.

3. Procédé selon l'une des revendications précédentes, dans lequel la coupe hydrocarbonée comprenant des composés ayant un point d'ébullition supérieur à 385°C issue de l'étape d) est au moins en partie recyclée à l'étape b).

4. Procédé selon l'une des revendications précédentes, comprenant une étape a0) de prétraitement de la charge, ladite étape de prétraitement étant mise en œuvre en amont de l'étape a) d'hydrogénation sélective optionnelle ou en amont de l'étape b) d'hydroconversion et comprend une étape de filtration et/ou une étape de lavage à l'eau et/ou une étape d'adsorption.

5. Procédé selon l'une des revendications précédentes, dans lequel l'effluent hydrocarboné liquide hydrotraité issu de l'étape f), tout ou partie, est envoyée vers une étape h) de vapocraquage réalisée dans au moins un four de pyrolyse à une température comprise entre 700 et 900°C et à une pression comprise entre 0,05 et 0,3 MPa relatif.

**6.** Procédé selon l'une des revendications précédentes, lequel comprend en outre une étape g) de recyclage dans laquelle une fraction de l'effluent hydrocarboné liquide hydrotraité issu de l'étape f) de séparation, est envoyée vers l'étape a) d'hydrogénation sélective optionnelle et/ou l'étape b) d'hydroconversion et/ou l'étape e) d'hydrotraitement.

**7.** Procédé selon l'une des revendications précédentes, dans lequel l'étape de séparation f) comprend un fractionnement permettant d'obtenir, outre un flux gazeux, une coupe naphta comprenant des composés ayant un point d'ébullition inférieur ou égal à 175°C, et une coupe diesel comprenant des composés ayant un point d'ébullition supérieur à 175°C et inférieur à 385°C.

**8.** Procédé selon la revendication précédente, lequel comprend en outre une étape e') d'hydrocraquage mise en œuvre dans une section réactionnelle d'hydrocraquage, mettant en œuvre au moins un lit fixe ayant n lits catalytiques, n étant un nombre entier supérieur ou égal à 1, comprenant chacun au moins un catalyseur d'hydrocraquage, ladite section réactionnelle d'hydrocraquage étant alimentée au moins par ledit effluent hydrotraité issu de l'étape e) et/ou par la coupe diesel comprenant des composés ayant un point d'ébullition supérieur à 175°C et inférieur à 385°C issue de l'étape f) et un flux gazeux comprenant de l'hydrogène, ladite section réactionnelle d'hydrocraquage étant mise en œuvre à une température entre 250 et 450°C, une pression partielle d'hydrogène entre 1,5 et 20,0 MPa abs. et une vitesse volumique horaire entre 0,1 et 10,0 $h^{-1}$, pour obtenir un effluent hydrocraqué qui est envoyé dans l'étape f) de séparation.

**9.** Procédé selon l'une des revendications précédentes, dans lequel l'étape séparation f) comprend en outre un fractionnement de la coupe naphta comprenant des composés ayant un point d'ébullition inférieur ou égal à 175°C en une coupe naphta légère comprenant des composés ayant un point d'ébullition inférieure à 80°C et une coupe naphta lourde comprenant des composés ayant un point d'ébullition entre 80 et 175°C.

**10.** Procédé selon la revendication 9, dans lequel au moins une partie de ladite coupe naphta lourde est envoyée vers un complexe aromatique comportant au moins une étape de reformage du naphta et/ou dans lequel au moins une partie de la coupe naphta légère est envoyée dans l'étape h) de vapocraquage.

**11.** Procédé selon l'une des revendications précédentes dans lequel ledit catalyseur d'hydrogénation sélective de l'étape a) comprend un support choisi parmi l'alumine, la silice, les silices-alumines, la magnésie, les argiles et leurs mélanges et une fonction hydro-déshydrogénante comprenant soit au moins un élément du groupe VIII et au moins un élément du groupe VIB, soit au moins un élément du groupe VIII.

**12.** Procédé selon l'une des revendications précédentes dans lequel lorsque l'étape b) est mise en œuvre en lit bouillonnant ou en lit mobile, ledit catalyseur d'hydroconversion de l'étape b) comprend un catalyseur supporté comprenant un métal du groupe VIII choisi dans le groupe formé par le Ni, Pd, Pt, Co, Rh et/ou Ru, optionnellement un métal du groupe VIB choisi dans le groupe Mo et/ou W, sur un support minéral amorphe choisi dans le groupe formé par l'alumine, la silice, les silices-alumines, la magnésie, les argiles et les mélanges d'au moins deux de ces minéraux, et lorsque l'étape b) est mis en œuvre en lit entrainé, ledit catalyseur d'hydroconversion de l'étape b) comprend un catalyseur dispersé contenant au moins un élément choisi dans le groupe formé par Mo, Fe, Ni, W, Co, V, Ru.

**13.** Procédé selon l'une des revendications précédentes dans lequel ledit catalyseur d'hydrotraitement de l'étape e) comprend un support choisi dans le groupe constitué par l'alumine, la silice, les silices-alumines, la magnésie, les argiles et leurs mélanges, et une fonction hydro-déshydrogénante comprenant au moins un élément du groupe VIII et/ou au moins un élément du groupe VIB.

**14.** Procédé selon les revendications 8 à 13, dans lequel ledit catalyseur d'hydrocraquage de l'étape e') comprend un support choisi parmi les alumines halogénées, les combinaisons d'oxydes de bore et d'aluminium, les silice-alumines amorphes et les zéolithes et une fonction hydro-déshydrogénante comprenant au moins un métal du groupe VIB choisi parmi le chrome, le molybdène et le tungstène, seul ou en mélange, et/ou au moins un métal du groupe VIII choisi parmi le fer, le cobalt, le nickel, le ruthénium, le rhodium, le palladium et le platine.

**15.** Procédé selon l'une des revendications précédentes, dans lequel la charge a les propriétés suivantes :

- une teneur en aromatiques comprise entre 0 et 90 % poids,
- une teneur en halogénés comprise entre 2 et 5000 ppm poids,
- une teneur en éléments métalliques comprise entre 10 et 10000 ppm poids,
- dont une teneur en élément fer comprise entre 0 et 100 ppm poids,

- une teneur en élément silicium comprise entre 0 et 1000 ppm poids.


**Patentansprüche**

1. Verfahren zur Behandlung eines Einsatzmaterials, das ein Pyrolyseöl aus Kunststoffen und/oder aus festen Sekundärbrennstoffen umfasst, umfassend:

   a) gegebenenfalls einen Schritt zur selektiven Hydrierung, der in einem Reaktionsabschnitt durchgeführt wird, dem zumindest das Einsatzmaterial und ein Gasstrom, der Wasserstoff umfasst, zugeführt wird, in Gegenwart von mindestens einem Katalysator für die selektive Hydrierung, bei einer Temperatur zwischen 100 und 280 °C, einem Wasserstoffpartialdruck zwischen 1,0 und 20,0 MPa abs. und einer Volumengeschwindigkeit pro Stunde zwischen 0,3 und 10,0 $h^{-1}$, um einen hydrierten Austrag zu erhalten;
   b) einen Hydrokonversionsschritt, der in einem Hydrokonversion-Reaktionsabschnitt unter Verwendung von mindestens einem Slurryreaktor, einem Reaktor mit Suspensionsbett und/oder Bewegtbett durchgeführt wird, der mindestens einen Hydrokonversionskatalysator umfasst, wobei dem Hydrokonversion-Reaktionsabschnitt zumindest das Einsatzmaterial oder der hydrierte Austrag aus Schritt a) und ein Gasstrom, der Wasserstoff umfasst, zugeführt wird, wobei der Hydrokonversion-Reaktionsabschnitt bei einer Temperatur zwischen 250 und 450 °C, einem Wasserstoffpartialdruck zwischen 1,0 und 20,0 MPa abs und einer Volumengeschwindigkeit pro Stunde zwischen 0,05 und 10,0 $h^{-1}$ betrieben wird, um einen hydrokonvertierten Austrag zu erhalten;
   c) einen Trennschritt, dem der hydrokonvertierte Austrag aus Schritt b) und eine wässrige Lösung zugeführt wird, wobei der Schritt bei einer Temperatur zwischen 50 und 450 °C durchgeführt wird, um mindestens einen Gasaustrag, einen wässrigen Austrag und einen Kohlenwasserstoffaustrag zu erhalten;
   d) einen Schritt zur Fraktionierung des gesamten oder eines Teils des Kohlenwasserstoffaustrags aus Schritt c), um mindestens einen Gasstrom, eine Kohlenwasserstofffraktion, die Verbindungen mit einem Siedepunkt kleiner gleich 385 °C umfasst, und eine Kohlenwasserstofffraktion zu erhalten, die Verbindungen mit einem Siedepunkt von über 385 °C umfasst,
   e) einen Hydrotreating-Schritt, der in einem Hydrotreating-Reaktionsabschnitt unter Verwendung von mindestens einem Festbettreaktor mit n Katalysatorbetten durchgeführt wird, wobei n eine Ganzzahl größer gleich 1 ist, das jeweils mindestens einen Hydrotreating-Katalysator umfasst, wobei dem Hydrotreating-Reaktionsabschnitt zumindest ein Teil der Kohlenwasserstofffraktion aus Schritt d), die Verbindungen mit einem Siedepunkt kleiner gleich 385 °C umfasst, und ein Gasstrom, der Wasserstoff umfasst, zugeführt wird, wobei der Hydrotreating-Reaktionsabschnitt bei einer Temperatur zwischen 250 und 430 °C, einem Wasserstoffpartialdruck zwischen 1,0 und 20,0 MPa abs. und einer Volumengeschwindigkeit pro Stunde zwischen 0,1 und 10,0 $h^{-1}$ betrieben wird, um einen per Hydrotreating behandelten Austrag zu erhalten;
   f) einen Trennschritt, dem der per Hydrotreating behandelte Austrag aus Schritt e) zugeführt wird, um mindestens einen Gasaustrag und einen per Hydrotreating behandelten flüssigen Kohlenwasserstoffaustrag zu erhalten.

2. Verfahren nach dem vorhergehenden Anspruch, das Schritt a) zur selektiven Hydrierung umfasst.

3. Verfahren nach einem der vorhergehenden Ansprüche, wobei die Kohlenwasserstofffraktion aus Schritt d), die Verbindungen mit einem Siedepunkt von über 385 °C umfasst, zumindest teilweise erneut Schritt b) zugeführt wird.

4. Verfahren nach einem der vorhergehenden Ansprüche, das einen Schritt a0) zur Vorbehandlung des Einsatzmaterials umfasst, wobei der Schritt zur Vorbehandlung vor dem optionalen Schritt a) zur selektiven Hydrierung oder vor Schritt b) zur Hydrokonversion durchgeführt wird und einen Filterschritt und/oder einen Waschschritt mit Wasser und/oder einen Adsorptionsschritt umfasst.

5. Verfahren nach einem der vorhergehenden Ansprüche, wobei der per Hydrotreating behandelte flüssige Kohlenwasserstoffaustrag aus Schritt f) vollständig oder teilweise zu einem Steamcracking-Schritt h) geleitet wird, der in mindestens einem Pyrolyseofen bei einer Temperatur zwischen 700 und 900 °C und bei einem Relativdruck zwischen 0,05 und 0,3 MPa erfolgt.

6. Verfahren nach einem der vorhergehenden Ansprüche, das ferner einen Rückführschritt g) umfasst, wobei ein Anteil des per Hydrotreating behandelten flüssigen Kohlenwasserstoffaustrags aus dem Trennschritt f) in den optionalen Schritt a) zur selektiven Hydrierung und/oder den Hydrokonversionsschritt b) und/oder den Hydrotreating-Schritt e) geleitet wird.

**7.** Verfahren nach einem der vorhergehenden Ansprüche, wobei der Trennschritt f) eine Fraktionierung umfasst, mit der sich abgesehen von einem Gasstrom eine Naphthafraktion, die Verbindungen mit einem Siedepunkt kleiner gleich 175 °C umfasst, und eine Dieselfraktion erhalten lässt, die Verbindungen mit einem Siedepunkt von über 175 °C und unter 385 °C umfasst.

**8.** Verfahren nach dem vorhergehenden Anspruch, das ferner einen Hydrocracking-Schritt e') umfasst, der in einem Hydrocracking-Reaktionsabschnitt unter Verwendung von mindestens einem Festbett mit n Katalysatorbetten durchgeführt wird, wobei n eine Ganzzahl größer gleich 1 ist, das jeweils mindestens einen Hydrocracking-Katalysator umfasst, wobei dem Hydrocracking-Reaktionsabschnitt zumindest der per Hydrotreating behandelte Austrag aus Schritt e) und/oder die Dieselfraktion aus Schritt f), die Verbindungen mit einem Siedepunkt von über 175 °C und unter 385 °C umfasst, und ein Gasstrom, der Wasserstoff umfasst, zugeführt wird, wobei der Hydrocracking-Reaktionsabschnitt bei einer Temperatur zwischen 250 und 450 °C, einem Wasserstoffpartialdruck zwischen 1,5 und 20,0 MPa abs. und einer Volumengeschwindigkeit pro Stunde zwischen 0,1 und 10,0 $h^{-1}$ betrieben wird, um einen per Hydrocracking behandelten Austrag zu erhalten, der in den Trennschritt f) geleitet wird.

**9.** Verfahren nach einem der vorhergehenden Ansprüche, wobei der Trennschritt f) ferner eine Fraktionierung der Naphthafraktion, die Verbindungen mit einem Siedepunkt kleiner gleich 175 °C umfasst, in eine leichte Naphthafraktion, die Verbindungen mit einem Siedepunkt von unter 80 °C umfasst, und eine schwere Naphthafraktion umfasst, die Verbindungen mit einem Siedepunkt zwischen 80 und 175 °C umfasst.

**10.** Verfahren nach Anspruch 9, wobei mindestens ein Teil der schweren Naphthafraktion zu einem Aromatenkomplex geleitet wird, der mindestens einen Schritt zum Reformieren von Naphtha aufweist, und/oder wobei mindestens ein Teil der leichten Naphthafraktion in den Steamcracking-Schritt h) geleitet wird.

**11.** Verfahren nach einem der vorhergehenden Ansprüche, wobei der Katalysator zur selektiven Hydrierung von Schritt a) einen Träger, der ausgewählt ist aus Aluminiumoxid, Siliciumdioxid, Siliciumdioxid-Aluminiumoxiden, Magnesiumoxid, Tonen und ihren Mischungen, sowie eine hydrierend-dehydrierende Funktion umfasst, die entweder mindestens ein Element aus Gruppe VIII und mindestens ein Element aus Gruppe VIB oder mindestens ein Element aus Gruppe VIII umfasst.

**12.** Verfahren nach einem der vorhergehenden Ansprüche, wobei, wenn Schritt b) im Slurry- oder im Bewegtbett durchgeführt wird, der Hydrokonversionskatalysator von Schritt b) einen geträgerten Katalysator umfasst, der ein Metall aus Gruppe VIII, das aus der Gruppe ausgewählt ist, die aus Ni, Pd, Pt, Co, Rh und/oder Ru gebildet ist, gegebenenfalls ein Metall aus Gruppe VIB, das aus der Gruppe Mo und/oder W ausgewählt ist, auf einem amorphen mineralischen Träger umfasst, der aus der Gruppe ausgewählt ist, die von Aluminiumoxid, Siliciumdioxid, Siliciumdioxid-Aluminiumoxiden, Magnesiumoxid, Tonen und Mischungen aus mindestens zwei dieser Mineralien gebildet wird, und wenn Schritt b) im Suspensionsbett durchgeführt wird, der Hydrokonversionskatalysator von Schritt b) einen dispersen Katalysator umfasst, der mindestens ein Element enthält, das aus der Gruppe ausgewählt ist, die von Mo, Fe, Ni, W, Co, V, Ru gebildet wird.

**13.** Verfahren nach einem der vorhergehenden Ansprüche, wobei der Hydrotreating-Katalysator von Schritt e) einen Träger, der ausgewählt ist aus der Gruppe, die aus Aluminiumoxid, Siliciumdioxid, Siliciumdioxid-Aluminiumoxiden, Magnesiumoxid, Tonen und ihren Mischungen besteht, sowie eine hydrierend-dehydrierende Funktion umfasst, die mindestens ein Element aus Gruppe VIII und/oder mindestens ein Element aus Gruppe VIB umfasst.

**14.** Verfahren nach den Ansprüchen 8 bis 13, wobei der Hydrocracking-Katalysator von Schritt e') einen Träger, der ausgewählt ist aus halogenierten Aluminiumoxiden, Kombinationen aus Bor- und Aluminiumoxiden, amorphen Siliciumdioxid-Aluminiumoxiden und Zeolithen, sowie eine hydrierend-dehydrierende Funktion umfasst, die mindestens ein Metall aus Gruppe VIB, das aus Chrom, Molybdän und Wolfram, allein oder gemischt, ausgewählt ist, und/oder mindestens ein Metall aus Gruppe VIII umfasst, das aus Eisen, Cobalt, Nickel, Ruthenium, Rhodium, Palladium und Platin ausgewählt ist.

**15.** Verfahren nach einem der vorhergehenden Ansprüche, wobei das Einsatzmaterial folgende Eigenschaften aufweist:

- einen Gehalt an Aromaten zwischen 0 und 90 Gewichts-%,
- einen Gehalt an halogenierten Verbindungen zwischen 2 und 5000 Masse-ppm,
- einen Gehalt an metallischen Elementen zwischen 10 und 10.000 Masse-ppm,
- wovon ein Gehalt am Element Eisen zwischen 0 und 100 Masse-ppm beträgt,

- einen Gehalt am Element Silicium zwischen 0 und 1000 Masse-ppm.

**Claims**

1. Process for treating a feedstock comprising a solid recovery fuel and/or plastics pyrolysis oil, comprising:

   a) optionally, a selective hydrogenation step performed in a reaction section fed at least with said feedstock and a gas stream comprising hydrogen, in the presence of at least one selective hydrogenation catalyst, at a temperature of between 100 and 280°C, a partial pressure of hydrogen of between 1.0 and 20.0 MPa abs. and an hourly space velocity of between 0.3 and 10.0 $h^{-1}$, to obtain a hydrogenated effluent;
   b) a hydroconversion step performed in a hydroconversion reaction section, using at least one ebullated-bed reactor, entrained-bed reactor or moving-bed reactor, comprising at least one hydroconversion catalyst, said hydroconversion reaction section being fed at least with said feedstock or with said hydrogenated effluent obtained on conclusion of step a) and a gas stream comprising hydrogen, said hydroconversion reaction section being operated at a temperature of between 250 and 450°C, a partial pressure of hydrogen of between 1.0 and 20.0 MPa abs. and an hourly space velocity of between 0.05 and 10.0 $h^{-1}$, to obtain a hydroconverted effluent;
   c) a separation step, fed with the hydroconverted effluent obtained from step b) and an aqueous solution, said step being performed at a temperature of between 50 and 450°C, to obtain at least one gaseous effluent, an aqueous effluent and a hydrocarbon effluent;
   d) a step of fractionating all or some of the hydrocarbon effluent obtained from step c), to obtain at least one gas stream, a hydrocarbon cut comprising compounds with a boiling point of less than or equal to 385°C and a hydrocarbon cut comprising compounds with a boiling point above 385°C,
   e) a hydrotreatment step performed in a hydrotreatment reaction section, using at least one fixed-bed reactor containing n catalytic beds, n being an integer greater than or equal to 1, each comprising at least one hydrotreatment catalyst, said hydrotreatment reaction section being fed with at least some of said hydrocarbon cut comprising compounds with a boiling point of less than or equal to 385°C obtained from step d) and a gas stream comprising hydrogen, said hydrotreatment reaction section being operated at a temperature of between 250 and 430°C, a partial pressure of hydrogen of between 1.0 and 20.0 MPa abs. and an hourly space velocity of between 0.1 and 10.0 $h^{-1}$, to obtain a hydrotreated effluent;
   f) a separation step, fed with the hydrotreated effluent obtained from step e) to obtain at least a gaseous effluent and a hydrotreated liquid hydrocarbon effluent.

2. Process according to the preceding claim, comprising said selective hydrogenation step a).

3. Process according to either of the preceding claims, in which the hydrocarbon cut comprising compounds with a boiling point above 385°C obtained from step d) is at least partly recycled into step b).

4. Process according to one of the preceding claims, comprising a step a0) of pretreating the feedstock, said pretreatment step being performed upstream of the optional selective hydrogenation step a) or upstream of the hydroconversion step b) and comprises a filtration step and/or a step of washing with water and/or an adsorption step.

5. Process according to one of the preceding claims, in which the hydrotreated liquid hydrocarbon effluent obtained from step f) is sent, totally or partly, into a steam cracking step h) performed in at least one pyrolysis furnace at a temperature of between 700 and 900°C and at a pressure of between 0.05 and 0.3 MPa relative.

6. Process according to one of the preceding claims, which also comprises a recycling step g) in which a fraction of the hydrotreated liquid hydrocarbon effluent obtained from the separation step f) is sent into the optional selective hydrogenation step a) and/or the hydroconversion step b) and/or the hydrotreatment step e) .

7. Process according to one of the preceding claims, in which the separation step f) comprises a fractionation making it possible to obtain, in addition to a gas stream, a naphtha cut comprising compounds with a boiling point of less than or equal to 175°C, and a diesel cut comprising compounds with a boiling point above 175°C and below 385°C.

8. Process according to the preceding claim, which also comprises a hydrocracking step e') performed in a hydrocracking reaction section, using at least one fixed bed containing n catalytic beds, n being an integer greater than or equal to 1, each comprising at least one hydrocracking catalyst, said hydrocracking reaction section being fed at least with said hydrotreated effluent obtained from step e) and/or with the diesel cut comprising compounds with a boiling point above 175°C and below 385°C obtained from step f) and a gas stream comprising hydrogen, said hydrocracking reaction section being operated at a temperature of between 250 and 450°C, a partial pressure of hydrogen of between 1.5 and 20.0 MPa abs. and an hourly space velocity of between 0.1 and 10.0 h$^{-1}$, to obtain a hydrocracked effluent which is sent into the separation step f).

9. Process according to one of the preceding claims, in which the separation step f) also comprises fractionation of the naphtha cut comprising compounds with a boiling point of less than or equal to 175°C into a light naphtha cut comprising compounds with a boiling point below 80°C and a heavy naphtha cut comprising compounds with a boiling point of between 80 and 175°C.

10. Process according to Claim 9, in which at least part of said heavy naphtha cut is sent to an aromatic complex including at least one naphtha reforming step and/or in which at least part of the light naphtha cut is sent into the steam cracking step h).

11. Process according to one of the preceding claims, in which said selective hydrogenation catalyst of step a) comprises a support chosen from alumina, silica, silica-aluminas, magnesia, clays and mixtures thereof and a hydro-dehydrogenating function comprising either at least one group VIII element and at least one group VIB element, or at least one group VIII element.

12. Process according to one of the preceding claims, in which, when step b) is performed in an ebullated bed or in a moving bed, said hydroconversion catalyst of step b) comprises a supported catalyst comprising a group VIII metal chosen from the group formed by Ni, Pd, Pt, Co, Rh and/or Ru, optionally a group VIB metal chosen from the group Mo and/or W, on an amorphous mineral support chosen from the group formed by alumina, silica, silica-aluminas, magnesia, clays and mixtures of at least two of these minerals, and when step b) is performed in an entrained bed, said hydroconversion catalyst of step b) comprises a dispersed catalyst containing at least one element chosen from the group formed by Mo, Fe, Ni, W, Co, V and Ru.

13. Process according to one of the preceding claims, in which said hydrotreatment catalyst of step e) comprises a support chosen from the group consisting of alumina, silica, silica-aluminas, magnesia, clays and mixtures thereof and a hydro-dehydrogenating function comprising at least one group VIII element and/or at least one group VIB element.

14. Process according to Claims 8 to 13, in which said hydrocracking catalyst of step e') comprises a support chosen from halogenated aluminas, combinations of boron and aluminium oxides, amorphous silica-aluminas and zeolites and a hydro-dehydrogenating function comprising at least one group VIB metal chosen from chromium, molybdenum and tungsten, alone or as a mixture, and/or at least one group VIII metal chosen from iron, cobalt, nickel, ruthenium, rhodium, palladium and platinum.

15. Process according to one of the preceding claims, in which the feedstock has the following properties:

   - a content of aromatic compounds of between 0 and 90% by weight,
   - a content of halogenated compounds of between 2 and 5000 ppm by weight,
   - a content of metallic elements of between 10 and 10 000 ppm by weight,
   - including a content of iron element of between 0 and 100 ppm by weight,
   - a content of silicon element of between 0 and 1000 ppm by weight.

**Figure 1**

## RÉFÉRENCES CITÉES DANS LA DESCRIPTION

**Documents brevets cités dans la description**

- WO 2018055555 A
- FR 2001758
- FR 2008108
- FR 2008106
- WO 2014001632 A
- FR 2681871
- FR 3051375
- US 2008177124 A
- EP 0113297 A
- EP 0113284 A
- US 5221656 A
- US 5827421 A
- US 7119045 B
- US 5622616 A
- US 5089463 A
- US 6589908 B
- US 4818743 A
- US 6332976 B
- CN 102051202
- US 2007080099 A

**Littérature non-brevet citée dans la description**

- CRC Handbook of Chemistry and Physics. CRC press, 2000
- *The Journal of the American Chemical Society,* 1938, vol. 6Q, 309
- **C. LÓPEZ-GARCÍA et al.** Near Infrared Monitoring of Low Conjugated Diolefins Content in Hydrotreated FCC Gasoline Streams. *Oil & Gas Science and Technology - Rev. IFP,* 2007, vol. 62 (1), 57-68